(19) **Europäisches Patentamt / European Patent Office / Office européen des brevets**

(11) **EP 3 883 654 B1**

(12) **EUROPEAN PATENT SPECIFICATION**

(45) Date of publication and mention
of the grant of the patent:
**07.06.2023   Bulletin 2023/23**

(21) Application number: **19805679.8**

(22) Date of filing: **20.11.2019**

(51) International Patent Classification (IPC):
**A61Q 17/00** (2006.01)        **A61K 8/06** (2006.01)
**A61K 8/34** (2006.01)        **A61K 8/25** (2006.01)
**A61K 8/73** (2006.01)

(52) Cooperative Patent Classification (CPC):
**A61Q 17/005; A61K 8/062; A61K 8/25; A61K 8/34; A61K 8/732;** A61K 2800/412; A61K 2800/52

(86) International application number:
**PCT/EP2019/081982**

(87) International publication number:
**WO 2020/104553 (28.05.2020 Gazette 2020/22)**

(54) **STABILIZED OIL-IN-WATER EMULSION**

STABILISIERTE ÖL-IN-WASSER-EMULSION

ÉMULSION D'HUILE DANS L'EAU STABILISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2018   EP 18207348**

(43) Date of publication of application:
**29.09.2021   Bulletin 2021/39**

(73) Proprietors:
- **Engblom, Johan**
  **224 80 Lund (SE)**
- **Ali, Abdullah**
  **274 37 Skurup (SE)**
- **Wahlgren, Marie**
  **232 52 Åkarp (SE)**
- **Anderson, Christopher**
  **587 29 Linköping (SE)**

(72) Inventors:
- **SJÖÖ, Malin**
  **244 33 KÄVLINGE (SE)**
- **ENGBLOM, Johan**
  **224 80 LUND (SE)**

- **ALI, Abdullah**
  **274 37 SKURUP (SE)**
- **WAHLGREN, Marie**
  **232 52 ÅKARP (SE)**
- **ANDERSON, Christopher**
  **587 29 LINKÖPING (SE)**

(74) Representative: **AWA Sweden AB**
**Box 5117**
**200 71 Malmö (SE)**

(56) References cited:
**EP-A1- 3 167 871        WO-A2-2012/081132**
**DE-A1- 10 238 649       US-A1- 2003 139 307**
**US-A1- 2004 241 120      US-A1- 2008 045 491**
**US-A1- 2009 226 498      US-A1- 2012 214 878**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to an alcohol based oil-in-water emulsion composition, in form of alcohol based oil-in-water pickering emulsion, to a method for preparing the same, to topical sanitizers, like hand sanitizers, and to a method for preparing the same.

BACKGROUND

Antiseptic soaps and topical formulations have become an essential part of personal hygiene. However, some people, for example people working as health-care and laboratory personnel, tend to suffer from dry skin, often resulting in red, chapped, and cracked skin. In more severe cases, they can develop dry skin diseases, like irritant contact dermatitis, due to frequent use of these products. Therefore, there is a need of more skin friendly products which can provide satisfying anti-septic results in combination with skin caring properties.

Commonly used hand sanitizers comprise an alcohol, and is in form of a solution or as a gel. The alcohol is often ethanol, isopropanol, and n-propanol, and can be combined with humectants like glycerol. It could also be desirable to include emollients like a mineral oil or a triglyceride oil. However, these additional components jeopardize the physical stability and/or do not provide the effect nor give the sensation as expected.

A moisturizing hand sanitizer is described in WO 2009/109870.

However, there is a need for a product giving less irritation and dryness. Stabilization of oil-in-water emulsions by addition of particles or granules is known by, for example, WO 2012/082065 A1. Herein the stabilizing particles are of starch.

There is a need to provide a product which is physically stable and which also provides an improved tactile perception and sensation than presently available products.

In US 2004241120 A1 it is disclosed a cosmetic composition comprising oil-in-water emulsion and solid particles. Therein it is also mentioned to provide pickering emulsions but without disclosing any means for its production. US 2009/226498 A1 discloses a moisturizing hand sanitizer comprising a high internal phase emulsion mixed with water and an alcohol. No alcohol based oil-in-water pickering emulsion is disclosed therein. EP3167871 discloses a Pickering emulsion comprising 25% ethanol in at least 40% aqueous phase, 25% rice oil, and 15% spherical polylactic acid powder particles.

SUMMARY

The present invention relates to an alcohol containing oil-in-water emulsion composition being stabilized with stabilizing particles as well as to topical sanitizer composition, like a hand sanitizing composition, comprising the said alcohol containing oil-in-water emulsion composition.

An aspect of the invention is a stabilized oil-in-water emulsion composition. The oil-in-water-emulsion composition comprises the following components:

a) at least one liquid continuous aqueous phase (X) comprising at least one linear or branched $C_1$-$C_4$ alcohol and water;
b) at least one dispersed fatty phase (Y), comprising at least one oil; and
c) stabilizing particles (Z), consisting of components selected from solid particles and soft gel particles. The emulsion composition comprises at least 20%, by weight, of an $C_1$-$C_4$ alcohol, or mixture thereof.

In one embodiment, the oil-in-water-emulsion composition comprises the following components:

a) at least one liquid continuous aqueous phase (X) comprising at least one linear or branched $C_1$-$C_4$ alcohol and water;
b) at least one dispersed fatty phase (Y), comprising at least one oil; and
c) stabilizing particles (Z), consisting of components selected from solid particles and soft gel particles. The emulsion composition comprises at least 35%, by weight, of an $C_1$-$C_4$ alcohol, or mixture thereof.

The oil-in-water emulsions are a surfactant free composition. Thus oil-in-water emulsions causing less, or no, skin irritation and dermatitis are provided.

The oil-in-water composition as described above comprises at least one dispersed fatty phase (Y) having a melting point below 50ºC, typically between 24 and 37ºC, for example melting point of about 30ºC, 32ºC, or 34ºC. In one embodiment the dispersed fatty phase (Y) has a melting point below 20oC, thus below room temperature.

Thus, the oil-in-water composition have a melting temperature being close to the temperature of the skin. The dispersed fatty phase (oil, or an emollient) shall have no, or at least very limited solubility in the continuous aqueous phase formed by one or more alcohols and water, for example in aqueous phase formed by ethanol and water.

An oil-in-water emulsion composition containing alcohol is hereby provided, more specifically, a topical sanitizing composition which have the capacity to kill bacteria, viruses, and fungii, in an amount determined by standards, such as European Standards (EN) further described below. An oil-in-water composition having these features, and after topical administration, shows good anti-microbial properties as it is possible to include an amount of alcohol which is enough to show antimicrobial effect. Further, a topical sanitizing composition being less irritating than previously known is provided with the invention. The tactile properties of the composition herein described are advantageous, as it is easy to spread, and is pleasant to have on the skin with a non-greasy or non-tacky after feel. The composition may include higher amounts of emollient without giving a greasy feeling. Also, the composition is non-tacky as the particles, like starch, may counteract a tacky feeling caused by humectants like glycerol.

The emulsion composition comprises the following:

a) 40-98% of at least one liquid continuous phase (X);
b) 1-40% of at least one dispersed fatty phase (Y); and
c) 0.25 - 20% of stabilizing particles (Z).

More specifically, the emulsion composition comprises the following:

a) 40-95% of at least one liquid continuous phase (X);
b) 4-40% of at least one dispersed fatty phase (Y); and
c) 0.25 - 20% of stabilizing particles (Z).

Typically, the composition comprises 45-80%, by weight, of at least one liquid continuous phase (X); for example it comprises of 50-70% of at least one liquid continuous phase.

In another embodiment, the emulsion composition comprises 85-95%, by weight, of at least one liquid continuous phase (X); typically 90-95%.

An aspect of the invention is an emulsion composition comprising 4-40%, by weight, of at least one dispersed fatty phase (Y); typically 20-30%, by weight. Another aspect of the invention is an emulsion comprising 1-10%, by weight, of at least one dispersed fatty phase (Y).

The emulsion composition does also comprise a component providing a stabilizing effect of the oil-in-water emulsion, thus the stabilizing particles. The stabilizing particles (Z) are present in an amount of 0.25-20%, by weight.

An aspect of the invention is an emulsion composition comprising between 40 and 80% of at least one liquid continuous phase; typically of 40-70% of at least one liquid continuous phase. The at least one liquid continous phase comprises an alcohol, or a mixture of alcohols, and water. The alcohol to be included in the emulsion is a monoalcohol, or a dialcohol.The alcohol may be selected from ethanol, isopropyl alcohol, or n-propanol. Ethanol is most often present in an amount of 70%, or above, in the aqueous solution; isopropanol and n-propanol are typically present in an amount of 60%, or above, in aqueous solution. Also mixtures of alcohols may be included in the at least one liquid continuous phase herein described. The at least one liquid continuous phase contains alcohol in a total amount 60-70%, or above. Ethanol, isopropanol, and n-propanol are also commonly used alcohols as antimicrobials in topical sanitizing products. They have an effect upon bacteria, yeast, and encapsulated virus like HIV, hepatitis B- anc C-viruses, and influenzaviruses. These alcohols have also shown some effect upon non-encapsulated viruses like calicivirus, hepatitis A-virus and adenovirus.

In one embodiment the oil-in-water emulsion composition as defined herein includes a ratio of b) at least one dispersed fatty phase (Y), and the c) stabilizing particles (Z), to be between 2.5:1 and 5:1, by weight. The oil-in-water emulsion composition herein described may comprise approximately 200-400 mg starch per 1 ml oil phase. The ratio between starch and oil has an effect upon the size of oil droplets, higher amount of starch results in smaller droplet diameter. WO 2012/082065 A1 describes the relationship between these parameters. Typically the ratio of b) at least one dispersed fatty phase (Y), and the c) a solid phase, is 3:1, when starch particles as described above are included in the emulsion.

An aspect of the invention is the oil-in-water emulsion composition comprising one or more additives selected from:

i) additional antimicrobial agent:
ii) a denaturizing agent;
iii) a thickener;

iv) humectants;
v) antipruritic agent;
vi) fragrances, essential oils, etc;
vii) propellants.

An aspect of the invention is an oil-in-water emulsion composition for topical (dermal) administration.

Another aspect of the invention is a topical sanitizing composition comprising the oil-in-water emulsion composition as defined herein. The topical sanitizing composition is in form of a cream; in form of a foam; in form of a gel; in form of a solution, typically a sprayable solution; in form of a lotion, or in form of wet wipes for hand sanitizing. For example, when the topical sanitizing composition is a hand sanitizer, the composition is typically in form of a solution, a lotion, or in form of wet wipes.

An aspect of the invention is to provide an alcohol containing oil-in-water emulsion composition, as well as a topical sanitizing composition, having an antimicrobial effect, including antibiotic, antiviral, and antifungal effect. Thus the composition has an effect upon Gram negative bacteria, Gram positive bacteria, yeast organisms, and viruses. For example, the oil-in-water emulsion and the products comprising the emulsion have an effect upon the following bacteria *Staphylococcus aureus,* including vancomycin-resistant *S.aureus* (MRSA), *Pseudomonas aeruginosa, Enterococcus hirae, Enterococcus faecium,* vancomycin-resistant *Enterococcus (VRE), Escherichia coli, Salmonella enterica, Serratia marcesens, Klebisella pneumonia, Clostridium difficile,* and *Aspergillus niger.* The composition may also have an effect on tuberculocidal bacteria. For example, the oil-in-water emulsion and the products comprising the emulsion have an effect upon yeast, for example upon *Candida albicans*. For example, the oil-in-water emulsion and the products comprising the emulsion have an effect upon enveloped viruses, including Norovirus. Also polyomavirus, Rotavirus, and Adenovirus are affected by the emulsion herein described.

An aspect of the invention is a method for preparing the oil-in-water emulsion composition as herein defined, as well as the topical sanitizing composition herein defined, comprising the following steps:

a) providing at least one liquid continuous aqueous phase (X) comprising at least one linear or branched $C_1$-$C_4$-alcohol;
b) providing at least one dispersed fatty phase (Y), comprising at least one oil;
c) providing stabilizing particles (Z);
d1) dispersing the stabilizing particles (Z) in the continuous aqueous phase (X) of a) during mixing, or
d2) dispersing the stabilizing particles (Z) in the dispersed fatty phase (Y);
e) adding the dispersed fatty phase (Y) to the mixture (X+Z) of d1) , or adding the continuous aqueous phase (X) to the mixture (Y+Z) of d2) during mixing, at a temperature below 45°C;
f) optionally, emulsification by further mixing.

Optionally, additives can be added during or after any of the steps a) to f) (step g).

Thus, a composition is herein provided, more specifically, an oil-in-water emulsion composition, and a topical sanitizing composition comprising the oil-in-water emulsion composition, which have the capacity to act as antibicrobial agent, thus to inactivate or kill bacteria, viruses, and fungii in an amount determined by standards.

Further, a hand sanitizing composition being less irritating than previously known compositions is provided by the invention.

DETAILED DESCRIPTION

The oil-in-water emulsion composition as defined above, as well as the topical sanitizing product is herein further defined and explained.

By the term "oil-in-water emulsion" it is herein meant an oil-in-water emulsion wherein the water phase includes a combination of one or more alcohol and water. Thus, the oil-in-water emulsion herein defined is an alcohol containing emulsion, an alcohol based oil-in-water emulsion. The emulsion comprises at least 20%, by weight, of alcohol in water, alternatively the emulsion comprises at least 35%, by weight, of alcohol in water.

Suitable alcohols for use in the product include any water-soluble alcohol known in the art. Typically, the alcohol is a short chain alcohol, such as "$C_1$-$C_6$ alcohols". By the term "$C_1$-$C_6$ alcohols" it meant any linear or branched alcohol having 1 to 6 carbon atoms, for example $C_1$-$C_6$ alcohols. Typically, the alcohol is a monoalcohol. Examples of suitable alcohols include methanol, ethanol, n-propanol, isopropanol, butanol, t-butanol, 2-butanol, pentanol, and hexanol, or combination thereof. More typically, the alcohol is ethanol, isopropanol, or n-propanol, or combination thereof.

The alcohol can be a short chain alcohol, for example alcohols comprising 1-4 carbon atoms (also denoted $C_1$-$C_4$-alcohols). The $C_1$-$C_4$-alcohols may be selected from monoalcohols and dialcohols. Typically the alcohol is a monoalcohol. Non-limiting examples of suitable monoalcohols include methanol, ethanol, propanol, isopropyl alcohol, butanol, t-butanol, 2-butanol. For example, the at least one liquid continuous aqueous phase is a mixture of ethanol and isopropyl alcohol, or a combination of 2-butanol together with ethanol and isopropanol, or n-propanol.

Non-limiting examples of dialcohols are propane-1,2-dialcohol, and butane-1,4-dialcohol.

The alcohol may also be a denatured alcohol.

Generally, for the oil-in-water emulsion as herein described, where the alcohol together with water forms the liquid continuous phase. It is important that the alcohol, or the mixture of alcohols, is completely soluble, or substantially complete soluble, in water.

The oil-in-water emulsion composition contains at least one dispersed fatty phase (Y), comprising at least one oil. The at least one dispersed fatty phase is selected from mineral oil, or from oils having biological origin, triglycerides, or mixtures thereof. For the oil-in-water emulsion composition as well as the topical sanitizing composition herein described the mineral oil is selected from petrolatum based oils, petrolatum, paraffin oil, vaseline, normal alkenes, like $C_5$-$C_{30}$ alkenes, or mixture thereof. The oil having biological origin may be selected from alpine apple seed oil, apricot kernel oil, argan oil, avocado oil, babassu oil, baobab oil, black seed oil, borage oil, broccoli seed oil, canola oil, castor oil, cocoa butter, coconut oil, cucumber oil, evening primrose oil, grapeseed oil, hazelnut oil, hemp seed oil, jojoba oil, kukui nut oil, macadamia oil, mango butter, marula oil, moringa oil, neem oil, olive oil, plum oil, pomegranate oil, prickly pear seed oil, rapeseed oil, raspberry seed oil, rosehip oil, safflower oil, sesame oil, shea butter, sun flower oil, sweet almond oil, and tea seed oil, and mixtures thereof. The list of oil having biological origin is not exhaustive.

The triglyceride may have saturated or unsaturated $C_6$-$C_{22}$ fatty acids, preferably of fatty acids with saturated or unsaturated $C_{10}$-$C_{22}$ fatty acids. The term "triglycerides of fatty acids equal or more than 6 carbon atoms" means herein triglycerides with fatty acids with equal or more 6 carbon atoms, being unsaturated or saturated. A triglyceride oil to be included in the oil-in-water emulsion may comprise substantial amounts of mono- and diglycerides. The triglyceride may comprise one type of fatty acid, or a mixture of fatty acids. Examples of fatty acids with equal or more 6 carbon atoms; preferably of fatty acids with $C_{10}$-$C_{22}$ fatty acids.

Examples of fatty acids with equal or more than 6 carbon atoms are caproic acid ($C_6$), caprylic acid ($C_8$), capric acid ($C_{10}$), lauric acid ($C_{12}$), myristic acid ($C_{14}$), palmitic acid ($C_{16}$), stearic acid ($C_{18}$), arachidic acid ($C_{20:0}$), behenic acid ($C_{22:0}$), myristoleic acid ($C_{14:1}$) (omega 5), palmitoleic acid ($C_{16:1}$) (omega 7), oleic acid ($C_{18:1}$) (omega 9), ricinoleic acid ($C_{18:1\ OH}$), 9-eicosenoic acid ($C_{20:1}$) (omega 11), erucic acid ($C_{22:1}$), Linoleic acid ($C_{18:2}$) (omega 6), linolenic acid ($C_{18:3}$) (omega 3+6), linolenic acid ($C_{18:3\ ALA}$) (omega 3), linolenic acid ($C_{18:3\ GLA}$) (omega 6), pucinic acid ($C_{18:3}$) (omega 5), stearidonic acid ($C_{18:4}$), or mixtures thereof.

The list of fatty acids to be included in the oil-in-water emulsion composition and in the topical sanitizing composition is not exhaustive.

For a topical sanitizing composition, the oil, or mixture of oils shall melt below 50ºC, typically below 40ºC, or around skin temperature (for example at 32ºC). If a mixture of triglycerides are included in the oil phase, it shall be a mixture of triglycerides that co-crystallize. For a topical sanitizing composition, the mixture of triglycerides shall melt below 50ºC, typically below 40ºC, or around skin temperature (for example at 32ºC).

The melting point of the triglyceride depends on the length of the fatty acids, usually higher for those having longer chain. The melting point does also depend on if the fatty acid is saturated or unsaturated. Triglycerides having saturated fatty acids have a higher melting point.

Depending on the components selected to be included in the oil-in-water emulsion composition, or in the hand sanitizing composition, it is important to consider the melting temperature of the oil, to avoid gelatinization of starch particles upon heating, and to avoid losing the alcohol included in the composition due to evaporation. For example, ethanol is boiling at 78ºC. Therefore, the tactile experience of the emulsion composition may be adjusted by selecting the fatty acids included in the triglycerides having a melting point of, or below, 50ºC.

The oil, or the triglyceride, can also be chosen to minimise the dissolution in the alcohol/water mixture. The dispersed fatty phase (oil, triglyceride, or an emollient) shall have no, or at least very limited solubility in the continuous aqueous phase formed by one or more alcohols and water, as herein described. Constructing a ternary phase diagram for the system (alcohol - water -oil) is a convenient method to identify these properties. For this specific aim it is desirable to minimize the one phase regions (to more or less pure alcohol-water and oil, respectively) and maximize the two phase region required for making an emulsion system.

The higher chain length the fatty acids in said triglyceride have, the less soluble aqueous mixtures with alcohol they are. To maintain a low melting point it is preferably to choose oils with unsaturated fatty acids. Further, generally speaking pure oils also have higher melting points than their miscible and co-crystallizing mixtures.

The higher chain length the fatty acids in said triglyceride have, the less soluble they are. To maintain a low melting point it is preferably to choose oils with unsaturated fatty acids.

Another aspect is that triglycerides may crystallize in different polymorphs, having different melting points. The most stable polymorphs is the beta-form, also having the highest melting point. However, the system of triglycerides can be designed to crystallize in other forms, i.e. alpha form and beta prim form, which result in an oil phase with lower melting point.

The emulsion composition of the invention shall comprise triglycerides with fatty acids having a melting point of, or below, 50°C.

For example, the emulsion composition may comprise tricaprin (C10) triglyceride having a melting point of 32°C.

The oil-in-water emulsion composition herein defined is a so-called pickering emulsion. The term "pickering emulsion" means herein an emulsion that is stabilized by solid particles, or granules. The stabilization is provided by that the solid particles are adsorbed onto the interface between the two phases, completely or partly. With the stabilization the oil-in-water emulsion resist changes in its properties over time. When the topical sanitizing composition comprising the emulsion is in form of a foam wherein the foam is stabilized by the solid particles, in the same way as in the emulsion

The stabilized oil-in-water emulsion composition as herein described comprises stabilizing particles being solid particles or soft-gel particles. The particles may consist of silica, starch, cellulose (for example cellulose granules, or cellulose nanocrystals (CNC), lipid (like solid lipid nanoparticles), protein (like aggregated proteins, protein adsorbed on particles), polymeric particles (e.g. polystyrene, chitosan), microgels, carbon, pigment (organic and inorganic), silver and gold particles, titania, iron oxides, calcium carbonate, clay, or latex. For example the particles may consist of silica, starch, cellulose, lipid, protein, polymeric particles (e.g. polystyrene, chitosan), microgels, calcium carbonate, or latex. Typically, the solid particles consist of silica, starch, cellulose, or latex.

The solid particles are selected to have a particle size of 0.005-20 $\mu$m, for example a particle size of 0.05-20 $\mu$m, or of 0.1-20 $\mu$m , or of 0.2-20 $\mu$m or of 0.1-10 $\mu$m, or of 0.2-10 $\mu$m, or of 0.5-3 $\mu$m. Typically, the solid particles have a size of 0.1-10 $\mu$m, or of 0.5-3 $\mu$m. Another example is solid particles of 0.5-2 $\mu$m . Another example, when the particles consists of starch they typically have a size of 0.2-20 $\mu$m; typically a size of 0.2-10 $\mu$m; typically between 0.2-5 $\mu$m; more typically between 0.2-4 $\mu$m, such as between 0.5-2 $\mu$m. When the stabilizing particle is of silica, the particle size is typically between 2 nm - 2 $\mu$m; more typically 20-200 nm, or 20-150 nm, like 3-50 nm, or 5-40 nm.

For example, the size of the particles is 5, 10, 20, 30, 50, 100, 200, 300, 400, 500 nm, 1, 2, 3, 4, 5, 6, 7, 8, 9, 10, 15, or 20 $\mu$m (described as Mode (Peak) distribution).

Examples of solid particles and soft-gel particles are those of polysaccharide particles, like cellulose and starch.The soft-gel particles can be further described as totally or partially swollen starch granules, or cellulose granules. The starch granules defined herein may be obtained from any botanical source. For example, the botanical source may be quinoa, rice, maize, amaranth, barley, immature sweet corn, rye, triticale, wheat, buckwheat, cattail, dropwort, durain, grain tef, oat, parsnip, small millet, wild rice, canary grass, cow cockle, dasheen pigweed, and taro including waxy and high amylose variaties of the above.

In an embodiment of the invention the oil-in-water emulsion composition the stabilizing particles (Z) may comprise starch particles to stabilize the emulsion. The starch particles or granules to be included in the composition are native or have been subjected to hydrophobic modification. Starch can be chemically modified by treatment with, for example, different alkenyl succinyl anhydrides, for example octenyl succinyl anhydride (OSA), which is approved for food applications at an added amount of up to 3% based on the dry weight of starch. Propenyl succinyl anhydride is another option for this application. The hydrophobic octenyl group and the carboxyl or sodium carboxylate group increased starches' ability to stabilize emulsions. It is also possible to make the starch particles or granules more hydrophobic by grafting with chemicals having a hydrophobic side chain, for instance, by fairly uniform surface, at least with respect to hydrophobicity, thus the starch particle/granule covered droplets have similar surface properties allows for a strong adsorption at the oil-in-water interface (a contact angle not too far from 90°) the particles when dispersed in the aqueous phase are also in a state of weak aggregation. In this case the steric particle-based barrier consists of more than a simple densely packed layer of starch granules at the droplet surface, but also extends as a disordered layer/network of granules between droplets, having the whole aggregated structure held together by attractive interparticle forces, thus creating a weak gel like structure of at least two phases, together with stabilizing particles, wherein the stabilizing particles, the solid or soft-gel particles, or at least a portion of it, are arranged at the interface between the at least two phases, e.g. at the interface between an oil phase and a water based phase, and thereby stabilizing the emulsion.

The starch particles to be included may be made more hydrophobic by physical modification, e.g. by dry heating or by other means, such as a change in pH, high pressure treatment, irradiation, or enzymes. Dry heating causes the starch particles/granule surface proteins to change character form hydrophilic to hydrophobic. An advantage of thermal modification is that no specific labeling is required when used in applications like in food, pharmaceuticals, cosmetics, and biocides. Furthermore, the hydrophobic alteration is explicitly occuring at the granule surface.

In another embodiment of the invention the stabilizing particles constitute the interface between the hydrophobic (Y) and hydrophilic phase (X).

When the stabilizing particles are selected to be solid particles or granules of starch, the starch can be obtained from any botanical source. For example, the starch granules have the capability to stabilize the oil-in-water emulsion compositions. The starch particles are abundant, relatively in-expensive, and may be obtained from many different botanical sources. There is a large natural variation regarding size, shape, and composition of the starch particles. Starch has an intrinsic nutritional value and is a non-allergenic source, in contrast to other common emulsifiers used in for example food, where emulsifiers originating from egg and soy are commonly used.

The starch may be obtained from the following biological sources: quinoa, rice, maize, tapioca, amaranth, barley, immature sweet corn, rye, triticale, wheat, buckwheat, cattail, dropwort, durain, grain tef, oat, parsnip, small millet, wild rice, canary grass, cow cockle, dasheen pigweed, and taro including waxy and high amylose variaties of the above. Typically, the starch particles originate from quinoa, rice, tapioca, amaranth, oat, and maize. Starch particles can be of natural size or size adjusted by chemical or enzymatic hydrolysis, milling, dissolution, or precipitation.

The stabilizing particles (Z) are added to the oil-in-water emulsion in an amount to correspond approximately 0.005-70%, by volume, of the total emulsion, typically the stabilizing particles (Z) are added in an amount corresponding 0.25-20%, by volume.

The amount of added stabilizing particles is also preferably determined by the coverage of the droplet and coverage should be more than 10%.

The oil-in-water emulsion composition as well as the hand sanitizing composition may comprise one or more additives, to form a topical sanitizing composition. Such suitable additives may be selected from antimicrobial agents, denaturizing agent, thickener, humectants, and propellants.

The antimicrobial agent (including antibiotics, antifungal agents as well as antiviral agents) can be selected from the list of approved substances under EU Regulation (528/2012) or substances that are currently being supported in the review programme for product type 1. (PT 1 Human hygiene), e.g., propanol, isopropanol, lactic acid, ethanol.

Denaturizing agent can be added to the emulsion composition, and can be selected from n-propanol, isopropyl alcohol, butanol, methyl ethyl ketone (MEK), Bitrex, or mixture thereof.

Thickener, typically selected from cellulose based compositions, typically selected from hydroxy propyl cellulose, xanthan gum, carbomer. The choice of thickener depends on two parameters: it shall be compatible with the aqueous phase of alcohol and water; and it shall not have too pronounced emulsifying properties (c.f. ampiphilic polymers), thus the thickener shall not compete with the starch for being on the oil/water interface. Examples of suitable thickeners are hydrophobically modified cellulose (e.g. hydroxypropyl cellulose, HPC, (Klucel), hydroxyethyl cellulose, HEC, (Natrosol), methyl cellulose), Xanthan gum, Guar gum and Carbopol.

The humectants, i.e. osmolytes, are compounds with a property for resembling the NMF in skin can be included, like amino acids and derivatives thereof, lactic acid, pyrrolidone carboxylic acid (PCA), sugars, and urea. Also, compounds added as humectants can also reveal additional features. One example is dexpanthenol, which stimulates intracellular protein and lipid synthesis, increases proliferation, and enhances epidermal differentiation. The oil-in-water emulsion composition may also comprise antipruritic agents, suitable to combat itching. Examples of such agents are glycine, glycyrrhetinic acid, polidocanol.

Also additives like fragrances, essential oils, can be included in the oil-in-water emulsion composition.

The lists of examples of different additives shall not be considered exhaustive.

The oil-in-water emulsion composition and the hand sanitizing composition herein described may also comprise one or more acids. By adding an acid to an alcohol containing composition the antimicrobial effect is improved. Examples of suitable acids are citric acid, phosphoric acid, uric acid, urocanic acid, peracetic acid, and lactic acid. The list of acids is not considered exhaustive.

Emulsions, like oil-in-water emulsions, are thermodynamically unstable, based on the thermodynamically unfavorable contact between water and oil molecules. Components of the one phase (e.g. the oil phase) may however dissolve or mix to a degree in the other phase (e.g. the water phase). To understand the behaviour of the composition a phase diagram is suitable employed, like ternary phase diagram. How parameters like temperature, pressure or components affects the system may then be determined, and the most preferred system can easily be selected.

By drawing a phase diagram for the components included in the oil-in-water emulsion composition selected it is possible to determine the optimum composition of the oil-in-water emulsion composition. With a ternary phase diagram for oil-in-ethanol-water systems it is possible to determine the properties and compositions where the oil is not soluble in the continuous liquid phase, and a two-phase system is achieved.

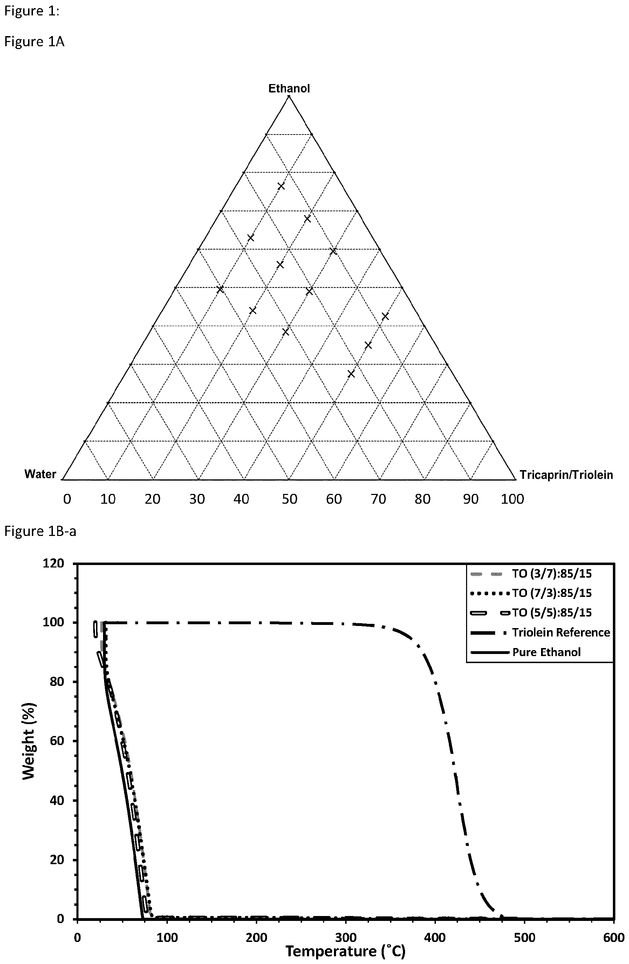

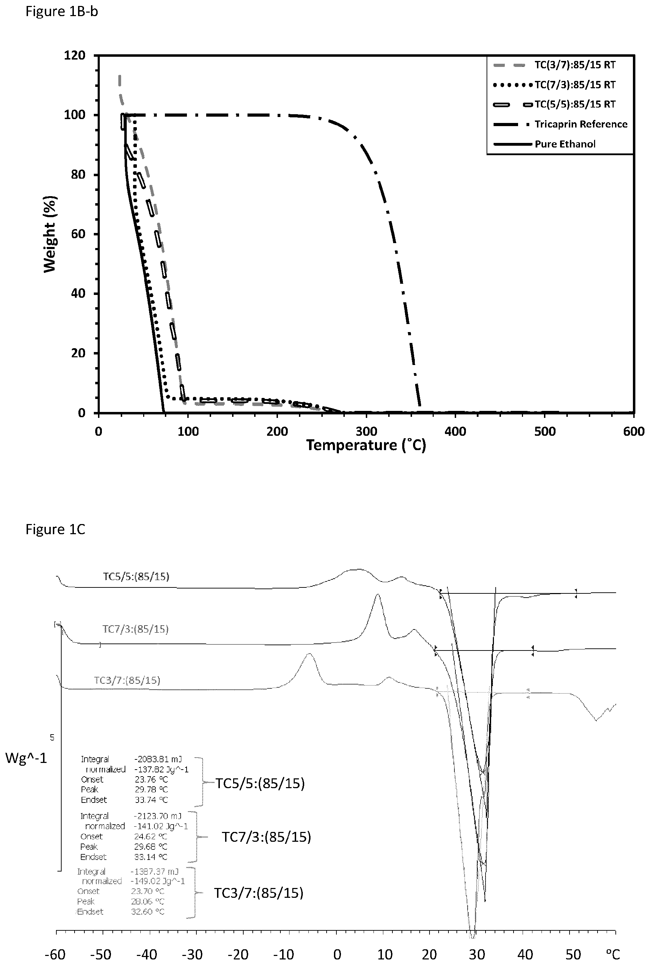

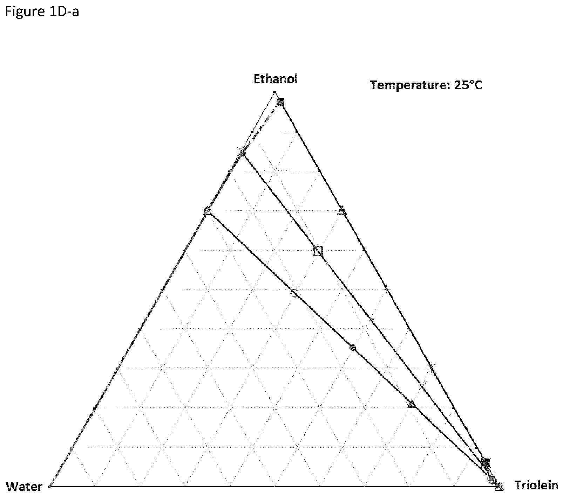

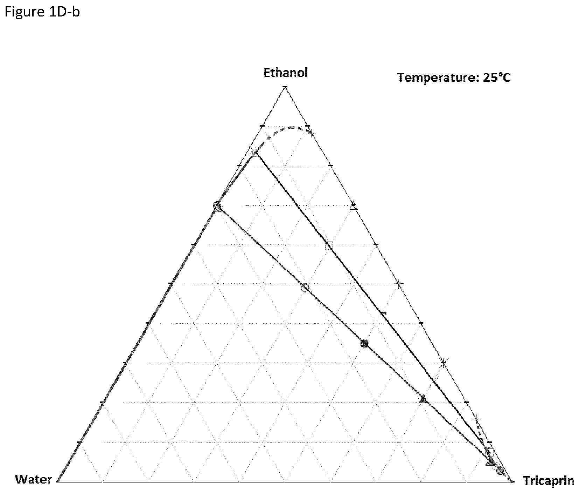

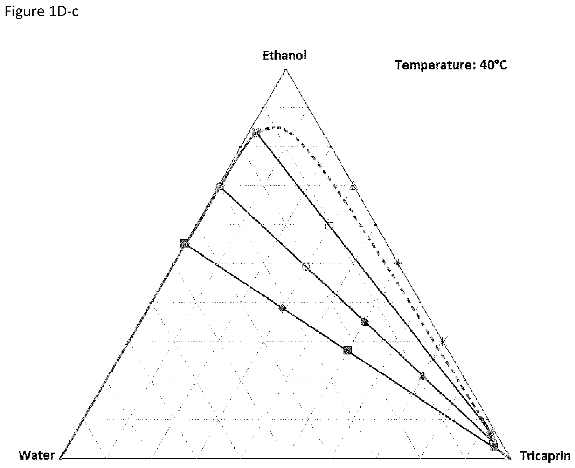

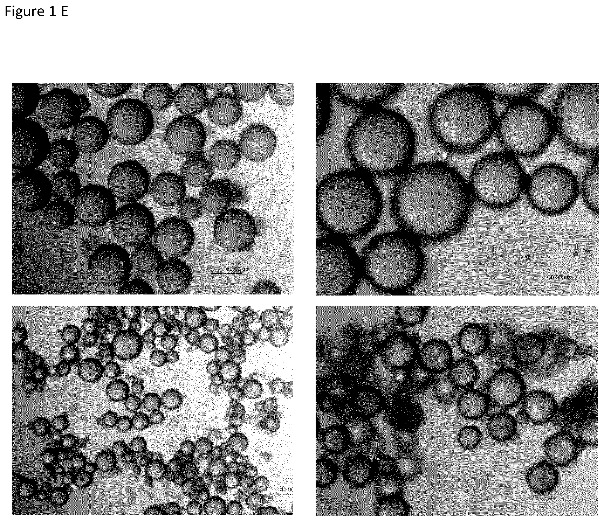

Ternary phase diagrams for triglyceride-ethanol-water systems has been drawn based on data presented in Table 1, 2 and 3, and calculated with lever rule, and the result is presented in Figure 1. Assumptions are made, such as that since the water content was not measured in the samples, it is assumed that ethanol/water ratio remains constant for all ethanol solutions.

Due to the advantageous properties of the oil-in-water emulsions described above the composition can be used in applications like topical sanitizing product. Topical sanitizing products are to kill microbes present on the skin, especially on the hands. Therefore, as the oil-in-water emulsion described above comprises an amount of alcohol, and an amount of emollient it is intended to be suitable to be included in a topical sanitizing product. The products comprising the oil-in-water emulsion as herein described are intended to include antiseptic forms of wound cream, ekzema cream, barrier cream, ointment, paste, wound gel, hydrogel, wound spray, foam spray, and wet wipes. Further, the product may be skin antiseptic forms of wound cream, ekzema cream, barrier cream, ointment, paste, wound gel, hydrogel, wound spray, foam spray, and wet wipes. Another product comprising the oil-in-water emulsion is skin disinfectant forms of wound cream, ekzema cream, barrier cream, ointment, paste, wound gel, hydrogel, wound spray, foam spray, and wet wipes.

An aspect of the invention is a method for preparing a topical sanitizing composition comprising the oil-in-water compisition as defined herein. The method comprises the following steps

a) providing at least one liquid continous aqueous phase (X) comprising at least one linear or branched $C_1$-$C_4$-alcohol;
b) providing at least one dispersed fatty phase (Y), comprising at least one oil;
c) providing stabilizing particles (Z);
d1) dispersing the stabilizing particles (Z) in the continuous aqueous phase (X) of a) during mixing, or
d2) dispersing the stabilizing particles (Z) in the dispersed fatty phase (Y);
e) adding the dispersed fatty phase (Y) to the mixture (X+Z) of d1), or adding the continuous aqueous phase (X) to the mixture (Y+Z) of d2), during mixing, at a temperature below 45°C;
f) optionally, emulsification by further mixing; and
g) optionally addition of additives during or after any of the steps a) to e).

For preparing a topical sanitizing composition in form of cream, lotion, a suitable thickener is selected as additive and added in step g).

For preparing a topical sanitizing composition in form of a sprayable form or a foam, a suitable propellant is added in step g).

For preparing wet wipes for hand sanitizing, the composition provided by step a) to g) is added to wipe substrate.

As a topical sanitizing product, the product shall provide a pleasant tactile sensation, providing an emollient film giving good perception of the product, and prevent dehydration of the skin due to the presence of the alcohol.

The hand sanitizing product as herein described is an alcohol based sanitizer. As a consequence of using this type of products is the dehydration of the skin connected to the evaporation of alcohols.

The present invention relates to alcohol based topical sanitizing compositions, including an oil as an emollient in a Pickering oil-in-water emulsion. The oil will act as an occlusive barrier to prevent further water evaporation from the skin after the alcohol is gone. Retained hydration of the skin together with smoothening of the skin by the emollient will result in increased friction. To determine this effect, tactile perception friction coefficients are identified.

In one aspect of the invention are topical sanitizing compositions, with a content of oil and alcohol plus water in an approximate ratio of about 30/70 (w/w) provided. Typically, they are in form of lotion or cream. These topical sanitizing compositions have the following general composition (amounts in %, by weight):

| | |
|---|---|
| Oil (emollient) | 24-28 |
| Particles | 8-9.5 |
| Alcohol | 32-44 |
| Denaturizing agent | 0-2 |
| Humectant | 0-5 |
| Thickener | 0.5-2 |
| Water | 15-25 |
| Sum: | 100 |

In one aspect of the invention are topical sanitizing compositions, with a content of oil and alcohol plus water in a ratio of 1/99 to 50/50 (w/w) provided.

The compositions obtained are semisolid, or liquid like. These topical sanitizing compositions have the following general composition (amounts in %, by weight):

| | |
|---|---|
| Oil (emollient) | 1-50 |
| Particles | 0.3-17 |
| Alcohol | 20-80 |
| Denaturizing agent: | 0-2 |
| Humectant | 0-10 |
| Thickener | 0.1-5 |
| Water | 0-40 |
| Sum: | 100 |

In one aspect of the invention are topical sanitizing compositions, with an oil and alcohol inwater, in a ratio of 1/99 - 10/90 (w/w) provided. The compositions obtained are liquid like, or in form of gel. These topical sanitizing compositions have the following general composition (amounts in %, by weight):

| | |
|---|---|
| Oil (emollient) (triglyceride) | 1.0-10 |
| Particle | 0.3-3.5 |
| Alcohol | 50-70 |
| Denaturizing agent: | 0-2 |
| Humectant: | 0-5 |
| Thickener | 0.2-1 |
| Water | 20-40 |
| Sum: | 100 |

In one aspect of the invention a method for preparing is provided. The method for preparing the oil-in-water emulsion composition as herein defined, as well as the topical sanitizing composition herein defined, comprising the following steps:

a) providing at least one liquid continous aqueous phase (X) comprising at least one linear or branched $C_1$-$C_4$ alcohol;
b) providing at least one dispersed fatty phase (Y), comprising at least one oil;
c) providing stabilizing particles (Z);
d1) dispersing the stabilizing particles (Z) in the continuous aqueous phase (X) of a) during mixing, or
d2) dispersing the stabilizing particles (Z) in the dispersed fatty phase (Y);
e) adding the dispersed fatty phase (Y) to the mixture (X+Z) of d1), or, adding the continuous aqueous phase (X) to the mixture (Y+X) of d2), during mixing, at a temperature below 45°C;
f) optionally, emulsification by further mixing.

Additives can be added during or after any of the different steps (a-f) (as step g) depending on the nature of the additive, like the solubility of the compound.

This final emulsification can take place using a high shear mixer (for example a Ika ULTRA-TURRAX, T 25, Germany) at 22000 rpm for 30-60 seconds based on batch volume and disperser (S25N 8G, S25N 10G or S25N 18G) The additives to be included in the oil-in-water composition may be added to the at least one dispersed fatty acid, or the at least one liquid continuous phase. For example, thickener and humectant may be added in the at least one liquid continuous phase, before dispersing the stabilizing particles.

Table 1

| Triglyceride:(Ethanol/H$_2$O) TG: EtOH/H$_2$O | TG:EtOH:H$_2$O (wt%) | Storage temperature (°C) | $w_{TG}$(EtOH) (wt%) | | $w_{TG}$(TG) (wt%) | |
|---|---|---|---|---|---|---|
| | | | 25°C | 40°C | 25°C | 40°C |
| Triolein | | | | | | |
| TO5:5(55/45) | 50:27.5:22.5 | 25 | - | - | - | - |
| TO5:5(70/30) | 50:35:15 | 25 | 0.1 | - | 97.7 | - |
| TO5:5(85/15) | 50:41.5:8.5 | 25 | 0.2 | - | 95.1 | - |
| TO5:5(100/0) | 50:50:0 | 25 | 2.8 | - | 94.9 | - |
| TO3:7(55/45) | 30:38.5:31.5 | 25 | - | - | - | - |
| TO3:7(70/30) | 30:49:21 | 25 | 0.1 | - | 90.9 | - |
| TO3:7(85/15) | 30:59.5:10.5 | 25 | 0.4 | - | 97.1 | - |
| TO3:7(100/0) | 30:70:0 | 25 | 3.3 | - | 92.5 | - |
| TO7:3(55/45) | 70:16.5:13.5 | 25 | - | - | - | - |
| TO7:3(70/30) | 70:21:0.9 | 25 | 0.1 | - | 96.8 | - |
| TO7:3(85/15) | 70:25:5.0 | 25 | 0.1 | - | 98.7 | - |
| TO7:3(100/0) | 70:30:0 | 25 | 1.6 | - | 94.4 | - |

(continued)

| Triglyceride:(Ethanol/H$_2$O) TG: EtOH/H$_2$O | TG:EtOH:H$_2$O (wt%) | Storage temperature (°C) | w$_{TG}$(EtOH) (wt%) | | w$_{TG}$(TG) (wt%) | |
|---|---|---|---|---|---|---|
| | | | 25°C | 40°C | 25°C | 40°C |
| Tricaprin | | | | | | |
| TC5:5(55/45) | 50:27.5:22.5 | 25, 40 | - | 0.1 | - | 95.1 |
| TC5:5(70/30) | 50:35:15 | 25, 40 | 0.2 | 0.5 | 95.9 | 94.0 |
| TC5:5(85/15) | 50:41.5:8.5 | 25, 40 | 1.9 | 2.2 | 89.9 | 89.8 |
| TC5:5(100/0) | 50:50:0 | 25, 40 | 11.8 | 1φ | 84.0 | 1φ |
| TC3:7(55/45) | 30:38.5:31.5 | 25, 40 | - | 0.1 | - | 91.3 |
| TC3:7(70/30) | 30:49:21 | 25, 40 | 1.0 | 0.6 | 94.4 | 94.4 |
| TC3:7(85/15) | 30:59.5:10.5 | 25, 40 | 2.2 | 4.0 | 91.4 | 89.6 |
| TC3:7(100/0) | 30:70:0 | 25, 40 | - | 1φ | - | 1φ |
| TC7:3(55/45) | 70:16.5:13.5 | 25, 40 | - | 0.1 | - | 100 |
| TC7:3(70/30) | 70:21:0.9 | 25, 40 | 0.5 | 0.2 | 92.6 | 99.2 |
| TC7:3(85/15) | 70:25:5.0 | 25, 40 | 1.1 | 1.0 | 90.4 | 94.5 |
| TC7:3(100/0) | 70:30:0 | 25, 40 | - | 1φ | - | 1φ |

**Table 1**. Phase boundaries in the ternary triglyceride-ethanol-water mixtures of tricaprin and triolein, respectively, determined from visual inspection and thermogravimetric analysis of compositions utilizing the lever rule. 1φ denotes a one-phase region, i.e., complete miscibility of the components at the given composition.

Table 2

| Excipients | Density (g/cm$^3$) at 20 °C |
|---|---|
| 55wt% Ethanol (aq) | 0.90 |
| 70wt% Ethanol (aq) | 0.87 |
| 85wt% Ethanol (aq) | 0.83 |
| 99.5wt% Ethanol (aq) | 0.79 |
| Tricaprin (s, β) | 1.05 |
| Triolein (l) | 0.91 |

**Table 2.** Densities for tricaprin, triolein and various mixtures of ethanol-water at 20°C[1].

Table 3

| Oil phase of TG:(EtOH/H$_2$O) sampled at 25°C | w$_{TG}$(TG) (wt%) | T$_{onset}$ (wt%) | T$_{endset}$ (°C) | ΔH$_{melting}$ (°C) |
|---|---|---|---|---|
| | (25°C) | (25°C) | (25°C) | (25°C) |
| Tricaprin | 100 | 31.9±0.8 (n=2) | 40.0±0.3 (n=2) | 163.8±5.6 (n=2) |
| TC$_{avg}$(55/45) | - | 25.9±1.9 (n=6) | 36.2±0.9 (n=6) | 134.8±16.1 (n=5,) |
| TC$_{avg}$(70/30) | 94.3±1.4 (n=3) | 25.0±1.5 (n=6) | 36.0±1.8 (n=6) | 141.5112.5 (n=6) |
| TC$_{avg}$(85/15) | 90.5±0.6 (n=3) | 24.6±1.7 (n=5) | 34.2±2.3 (n=5) | 149.4±5.7 (n=5) |
| TC$_{avg}$(100/0) | 83.99 (n=1) | 22.1±0.7 (n=5) | 31.9±3.1 (n=5) | 128.8±12.9 (n=5) |

(continued)

| Oil phase of TG:(EtOH/H$_2$O) sampled at 25°C | w$_{TG}$(TG) (wt%) | T$_{onset}$ (wt%) | T$_{endset}$ (°C) | ΔH$_{melting}$ (°C) |
|---|---|---|---|---|
| | (25°C) | (25°C) | (25°C) | (25°C) |
| | | | | |
| Triolein | 100 | -17.3±0.1(n=2) | -2.8±0 (n=2) | 60.5±1.2(n=2) |
| TO:55/45$_{avg}$ | - | -18.6±0.9 (n=2) | -0.27±0.3 (n=2) | 74.55±1.6(n=2) |
| TO:70/30$_{avg}$ | 95.1±3.0 (n=3) | -18.0±0.4 (n=3) | -0.55±0.8 (n=3) | 75.4±1.9(n=3) |
| TO:85/15$_{avg}$ | 97.0±1.5 (n=3) | - 18.0±0.08 (n=3) | -0.63±1.4 (n=3) | 69.8±1.7(n=3) |
| TO:100/0$_{avg}$ | 93.9±1.0 (n=3) | - 17.8±0.14 (n=3) | -1.50±1.0 (n=3) | 67.7±0.9(n=3) |

**Table 3**. Thermotropic data on melting (T$_{onset}$ , T$_{endset}$ and ΔH$_{melting}$) of the oil phase in the triglyceride/ethanol/water system separated from excess ethanol/water at 25°C.

By the invention it has been shown that it is possible to improve the stability of an oil-in-water emulsion composition. By selecting components having similar densities, the stability if the oil-in-water emulsion composition can be affected. For example, an oil-in-water emulsion composition comprising 55% ethanol (aq) and triolein, can have a shelf life stability of about 2 years.

Further, it is an advantage to minimize the amount of oil residue in the alcohol/water phase. The oil solubility in the alcohol can be adjusted by selecting chain length of the fatty acid in the triglyceride oil, as well as the degree of saturation of the fatty acid. For example, oil solubility in an ethanol/water solution can be minimized by choosing an oil with longer hydrocarbon chain. But, as the melting temperature of the oil, is an important parameter for the final product, it is possible to keep the melting temperature of the oil phase within the stipulated range by choosing an oil also having unsaturated hydrocarbon chains

Description of the Figures

Figure 1:

Figure 1A. The composition of the Pickering formulations prepared with tricaprin and triolein are plotted in the ternary phase diagram. 400 mg quinoa starch/mL oil was added as an emulsifier.

Figure 1B:a-b. An example of thermogravimetric analysis (TGA) graphs for ethanol/water fractions (85/15, by weight) from triolein-(ethanol/water) mixtures (upper plot- a) and tricaprin-(ethanol/water) mixtures (lower plot-b). TGA graphs for pure ethanol, pure triolein and pure tricaprin are included as reference. Further, the graphs show a much smaller oil residue in the ethanol/water phase when using triolein, compared to tricaprin. Evidently, oil solubility in an ethanol/water solution can be minimized by choosing an oil with longer hydrocabon chains. The melting point of the oil can be kept with in the here stipulated range by choosing an oil also having unsaturated hydrocabon chains.

Figure 1C. As an example, thermograms obtained from differential scanning calorimetry (DSC) measurements on tricaprin fractions from tricaprin-(ethanol/water (85/15, by weight)) mixtures, comprising 30, 50 and 70wt% tricaprin. The thermograms show the melting behaviour of the individual samples.

Figure 1D:a-c. Ternary phase diagrams for triolein-ethanol-water at room temperature (top - a), tricaprin-ethanol-water at room temperature (middle - b) and tricaprin-ethanol-water stored at 40°C (bottom- c). The phase diagrams were drawn based on the compositions of the respective fractions from phase separated triglyceride-ethanol-water samples, using TGA and the lever rule. The red symbols represent the compositions of individual samples prepared. The data points determined from phase separated samples, reflecting the phase boundaries, have the same symbols as the original samples but a different color.

Figure 1E. Examples of two Pickering emulsions. Micrographs of diluted (5x) o/w-emulsions comprising 28wt% triolein, 400mg starch per ml oil and 60wt% continous phase, where the continuous phase comprises 58wt% ethanol in water (upper) and pure water (lower), respectively. With 10x magnification (left) and 20x magnification (right).

Figure 2:

Figure 2A.
Average friction coefficients measured on hydrated VITRO-SKIN@ (IMS Inc, USA) after application of a traditional ethanol-based handsanitizer (Sterisol® Handdesinfektion Etanol (Sterisol AB, Sweden)) and two water-based Pickering emulsions containing tricaprin (PemTC-H2O, C10:0) and triolein (PemTO-H2O, C18:1), respectively. Both Pickering emulsions comprised 30wt% triglyceride (C10:0), 10wt% modified quinoa starch and 60wt% water. Finger friction measurements were also performed on VITRO-SKIN® alone as a reference accounting for effects of dehydration of the substrate over time. While the traditional handsanitizer gave high friction coefficients during application, they rapidly returned to the reference value (i.e that of the bare surface) as a result of ethanol evaporation. For both Pickering emulsions the friction coefficients increase from initial low values to reach a stable platau within the first 2.5 minutes, most probalby due to formation of a lipid film on the skin. No difference could be observed from changing the oil from tricaprin to triolein.

Figure 2B.
Average friction coefficients measured on hydrated VITRO-SKIN® (IMS Inc, USA) after application of two alcohol-based Pickering emulsions comprising 30wt% tricaprin (C10:0), 10wt% modified quinoa starch and 60wt% ethanol solution (55wt% (PemTC-55EtOH) and 70wt% (PemTC-70EtOH) ethanol in water) and compared to a water-based Pickering emulsion, based on the same recipee with 60wt% water (PemTC-H2O). Finger friction measurements were also performed on VITRO-SKIN® alone as a reference accounting for effects of dehydration of the substrate over time. The results for emulsion treated skin show that during the evaporation of water and ethanol, the remaining lipid film on the skin occludes and smoothens the skin resulting in higher friction coefficients. No difference in tactile perception (i.e. friction) could be observed from replacing parts of the water with increasing amounts of alcohol.

Figure 2C:
Average friction coefficients measured on hydrated VITRO-SKIN® (IMS Inc, USA) after application of two alcohol-based Pickering emulsions comprising 30wt% triolein (C18:1), 10wt% modified quinoa starch and 60wt% ethanol solution (55wt% (PemTO-55EtOH) and 70wt% (PemTO-70EtOH) ethanol in water) and compared to a water-based Pickering emulsion, based on the same recipee with 60wt% water (PemTO-H2O). Finger friction measurements were also performed on VITRO-SKIN® alone as a reference accounting for effects of dehydration of the substrate over time. The results for emulsion treated skin show that during the evaporation of water, the remaining lipid film on the skin occludes and smoothens the skin resulting in higher friction coefficients. No difference in tactile perception (i.e. friction) could be observed from changing the oil from tricaprin to triolein.

Figure 2D:
Average friction coefficients measured on hydrated VITRO-SKIN® (IMS Inc, USA) after application of two commercial reference products, a thickened solution (tilted lines) and an alcohol gel (horizontal lines), and two alcohol-based Pickering formulations in form of a 5wt% emollient hand sanitizing gel (tiles) and 28wt% cream (emollient antimicrobial cream) (black dots). Formulations of the current invention maintains higher friction coefficients over longer periods in comparison to reference commercial products.

Figure 3
Sensory profile of a Pickering formulation (A), comprising 20% oil in ethanol/water 70/30 (by weight), and two classical hand sanitizers, Sterisol® Handdesinfektion Etanol (Sterisol AB, Sweden) (B) (active ingredient: Ethanol (70 % w/w), Propane-2-ol (10 % w/w), Further ingredients: Aqua, Glycerin (humectant), t-Butyl Alcohol (denaturating agent), Carbomer (part of a thickener system) Aminomethyl Propanol (buffer), and 'DAX Clinical Handdesinfektion' (CCS Healthcare AB, Sweden) (C) (Ethanol (about 68-70%), Aqua, 2-propanol, Glycerin, Caprylyl glycol, t-butanol). The main differences between the Pickering formulation and the two classical hand sanitizers were related to the afterfeel, where the test panel experienced a dryer, less smooth and smaller residual coating, despite the fact that a Pickering formulation contains more non-volatile excipients which will remain on the skin after application.

EXAMPLE

By way of examples, and not limitation, the following examples identify a variety of oil-in-water emulsions and hand sanitizing compositions pursuant to embodiments of the present invention.

Oil-in-water emulsions comprising different components and corresponding topical sanitizing compositions are shown in the following examples.

The compositions are then analysed and tested regarding physical stability and tactile sensation.

Material

Ethanol (99.8%) was purchased from VWR Chemicals (Stockholm, Sweden), while tricaprin (Captex 1000, 98.5% purity) and triolein (Captex GTO, 90.2% purity) were obtained from Abitec Corporation (Janesville, USA). Glycerol was purchased from Sigma-Aldrich (Stockholm, Sweden).

Modified quinoa starch, used as Pickering particles, was provided by Speximo AB (Lund, Sweden), and prepared according to WO2012/082065.

Ultrahigh quality (UHQ) water, purified at 25°C by Elgastat UHQ II Model UHQ-PS-MK3 (Elga Ltd., High Wycombe, Bucks, UK) was used throughout the study. The artificial skin (i.e. Vitro-Skin®) used in friction measurements was purchased from IMS Inc. (USA). Two commercially available ethanol based hand sanitizers (Hand Desinfect Ethanol (Sterisol AB, Sweden) and DAX Clinical Handdesinfektion (CCS Healthcare AB, Sweden)) were sourced from a local pharmacy.

Methods

*Example 1A: Sample preparation of oil-ethanol-water mixtures, and visual evaluation of their mixing behviour.*

Samples were prepared by mixing ethanol-water solutions, comprising 55wt%, 70wt%, 85wt% and pure (99.8wt%) ethanol, with liquid triglycerides (i.e., tricaprin at 40°C or triolein at room temperature) in the following triglyceride-ethanol solution weight ratios: 30:70, 50:50 and 70:30 (For simplicity, ethanol-water solutions will be hereafter referred to as ethanol solutions together with the specific concentration). The samples were then left to equilibrate in the dark in flame-sealed glass ampoules at 25 °C and 40 °C reflecting relevant stability study temperatures. The sample set is summarized in table 1. All samples were photographed directly after mixing, 24 hours after preparation and weekly during storage for 8 (50:50 samples) and 2 weeks (30:70 and 70:30 samples). Macroscopic phase behavior, including phase separation, was noted and ampoules were then opened to extract individual phases for further analysis. The results are illustrated in phase diagrams provided as figure 1D.

*Example 1B: Thermogravimetric analysis.*

Thermogravimetric analysis (TGA) was primarily used to determine the amount of triglyceride dissolved into the ethanol-water phase in samples suffering phase separation (Figure 1B). The weight loss of the samples studied was determined as a function of time and temperature using a TGA Q500 (TA Instruments, New Castle, USA). By combining the measured weight content remaining at a certain temperature and known boiling points for each constituent, TGA could be used to determine the content of each constituent in the studied samples. Samples were transferred with a plastic pipette to a platinum pan. The starting temperature was 25°C (RT) and samples were held isothermal for 1 minute, followed by a 10°C/minute ramp until 600°C. At 600°C the temperature was held isothermal for 5 minutes before stopping the measurement.

*Example 1C: Differential scanning calorimetry (DSC).*

The thermal phase behavior of the triglyceride fraction in samples suffering phase separation was studied by DSC (DSC 1, Mettler Toledo, Greifensee, Switzerland) (Figure 1C). Extracted fractions (10-15 mg) were placed in hermetically sealed 40 $\mu$l aluminum pans and placed in the DSC-instrument. The samples were first cooled down (10°C/min) to -60°C, kept isothermally for 2 minutes at this temperature and then heated (10°C/min) to 60°C and held isothermally for 2 minutes. Another round of cooling to -60°C, 2 minutes isothermal hold time followed by heating to 60°C was performed to evaluate any changes in melting temperature upon rapid cooling of the samples. Nitrogen was used as carrier gas at 80 ml/min and calibration for heat flow and temperature was done with indium ($T_m$=156.6°C; $\Delta$H=28.45 J/g).

For comparison, the commercial triglycerides (C18:1) and (C10:0) were also analyzed as received before mixing.

*Example 1D: Preparation of Pickering formulations.*

Pickering formulations were prepared with two alternative triglycerides, tricaprin or triolein, as the dispersed oil phase and ethanol solutions as the continuous polar phase. Modified quinoa starch was added as 400 mg starch per ml oil in all emulsions for particle stabilization. The compositions were chosen within the two-phase region of the ternary triglyceride-ethanol-water phase diagrams (Figure 1D) in order to test whether Pickering emulsions could be formed with this system. For comparison, water-based Pickering emulsions were prepared with the same oil content.

In Figure 1A, the oil/ethanol/water ratios of the potential/tentative pickering formulations are shown on a ternary diagram. The primary formulations comprised 30wt% oil phase and 60wt% polar phase mixed and emulsified with 10wt% modified quinoa starch. The ethanol concentration of the hydrophilic phase then varied between 55 and 70wt% (corresponding to 33-42wt% in the complete formulation). Starch (0.8 g) was added and vortexed with the polar phase (4.2g) for 30 seconds before adding the triglycerides (1.8 g) and vortexing for another 30 seconds followed by emulsification with a high shear mixer (Polytron PT3000) for 1 minute at 22000 rpm. The test tubes were photographed to follow the emulsion stability with time.

*Example 1E: Optical microscopy.*

Pickering formulations were also analyzed by optical light microscopy to evaluate emulsion stability. 1-2 drops of formulation was diluted (5x) with water and spread on a glass-slide without using a cover glass(Figure 1E).

Example 1F: *Ex vivo Perception Testing utilizing ForceBoard™ with VITRO-SKIN®.*

Tactile friction measurements were performed using a ForceBoard™(Industrial Dynamics Sweden AB, Järfälla, Sweden), equipped with both a horizontal and tangential load cell, consisting of strain gauges in a Wheatstone bridge configuration. A mechanical load results in voltage changes that are proportional to the applied load. The friction force (F) and applied load (L) were continuously recorded, with a sampling rate of 100 Hz, as a finger interrogated the model skin surface by moving the index finger back and forth, and the friction coefficients ($\mu$) were calculated as the ratio of the friction force and load according to:

$$\mu = \frac{F}{L}$$

VITRO-SKIN@ (IMS Inc., USA) was used as a model skin as it mimics human skin surface properties in terms of topography, pH, elasticity, surface tension and ionic strength [www.ims-usa.com/vitro-skin-substrates/vitro-skin/]. The model skin was cut into pieces of 2.5 cm x 5 cm and placed in a desiccator on the internal shelf above a beaker with a mixture of 85wt% water and 15wt% glycerol for 16-24 hours before use. This allowed for reproducible hydration of the skin samples prior to friction measurements[2]. Prior to each measurement, the model skin was taken from the desiccator and weighed before attachment to the ForceBoard™ with double-adhesive tape. The ForceBoard™ was heated to 32 °C by using a heating block. A finger friction measurement with 10 strokes was recorded without any formulation before each measurement series as a reference measurement on the model skin and clean finger. Approximately 6-7 mg/cm[2] of emulsion was applied to the model skin and the friction measurement was started by using the index finger, inclined at about 30°, to spread the emulsion by stroking forward (away from the body) and back (towards the body) 10-12 times over a sample area of 7.5 cm[2]. The friction was measured again after 2.5 minutes, 5 minutes and 10 minutes without washing the index finger.

However, between each experiment the finger was cleaned with soap, thoroughly rinsed with water and allowed to dry for 3 minutes before next experiment. The moisture content of the finger was also measured using a Corneometer (CM825, Courage Khazaka Electronic GmbH) prior to each test. The measurement time period was based on evaporation experiments where > 50wt% of the hydrophilic phase evaporated within 10 minutes in all formulations. All experiments were performed in triplicate and in controlled environment (T=21°C and RH=50%). The load, stroking distance and inclination of the finger were all controlled. To minimise the variability in the measurements further, the same experimenter performed all measurements with the same finger.

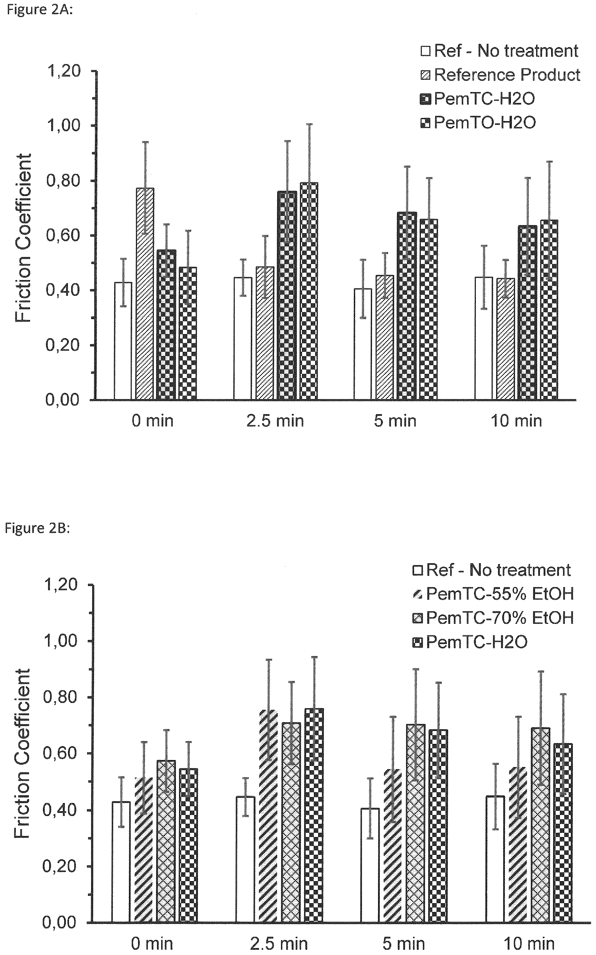

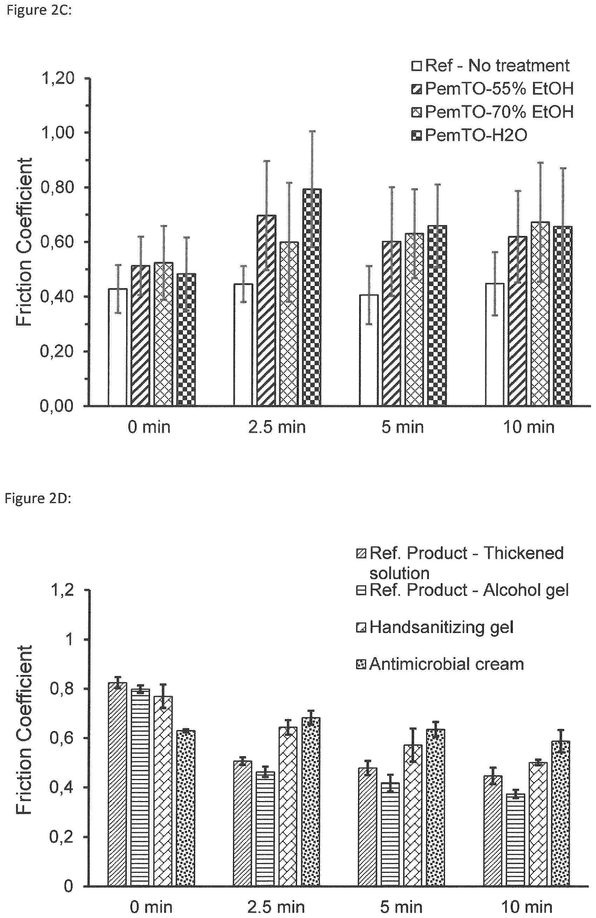

There is a clear and persistent increase in friction coefficient for the Pickering emulsions compared to the reference product (a traditional ethanol based hand disinfectant) and the reference on VITRO-SKIN@ alone (Figure 2). The friction coefficient for the reference product was highest upon application when the skin was hydrated by the solution. However, it was largely reduced after 2.5 minutes and after 10 minutes, it was similar to the reference (on skin model) resembling dry skin. Contrary, the friction coefficients caused by both Pickering emulsions were similar to the reference (i.e. VITRO-SKIN@ alone) upon application, but increased within 2.5 minutes and remained high after 10 minutes. The results for emulsion treated skin suggest that during the evaporation of water, the remaining oil film on the skin occludes and smoothens the skin resulting in higher friction coefficients. This shows a clear benefit of using water-based Pickering formulations over the ethanol-based reference product. The friction coefficients for ethanol-based Pickering formulations with triolein and tricaprin as emollients are shown in figures 2B and 2C. The results show similar behavior for both types of formulations, an initial increase in friction coefficient upon application, probably caused by the ethanol hydrating the skin before evaporating, followed by a further increase in friction coefficient within 2.5 minutes caused by the oil residue, which more or less remains the same over the measurement period. The friction coefficients remain higher than that of the reference (i.e. VITRO-SKIN@ alone) after drying the formulations for 10 minutes. This type of prolonged high friction on skin caused by an oil containing formulation would be beneficial in terms of tactile perception, where, the consumer experience the skin as soft and pleasant after the alcohol had killed the germs and evaporated. The results do not reveal any difference between formulations comprising an emollient that is liquid or solid (e.g. triolein vs tricaprin) at room temperature.

The results show a clear benefit from using Pickering-emulsion-based formulations in terms of tactile perception over conventional alcohol-based hand sanitizers. In addition, ethanol-based Pickering formulations comprising higher amounts of emollients (e.g. about 30wt% triglycerides) show a great potential to be a better alternative to conventional hand sanitizers in terms of skin dehydration and tactile perception of the skin

Example 2 - Oil-in-water emulsions as pickering formulations

Ternary diagrams for triolein and tricaprin were used to prepare oil-in-water emulsion composition according to the invention (Figure 1D). Herein the liquid continuous aqueous phase and the dispersed fatty phase were not miscible. Compositions were prepared with tricaprin and triolein and stored at room temperature. The primary compositions, comprising 30wt% lipid (oil), 60wt% ethanol, were white milky emulsions with a thin ethanol/water layer on the top.

Analysis with microscopy, compositions comprising 28wt% triglyceride (tricaprin/triolein), 12wt% quinoa starch and 60wt% of aqueous ethanol (58/42wt% ethanol/water) for triolein and tricaprin respectively, confirmed particle based stabilization where the starch particles cover the oil droplets (Figure 1E).

Example 3 - Emollient hand sanitizer

Compositions with oil/(alcohol+water) of ratio 30/70, in form of lotion, cream are prepared according to the method as described in herein The hand sanitizing composition as described below is prepared according to the method for preparing herein described.

All the aqueous components are mixed together while the oil soluble components are mixed together in a separate container. The thickener is dispersed in the aqueous phase during mixing until it is fully dispersed, before addition of quinoa starch particles during mixing. The oil phase is finally added to the aqueous blend during mixing followed by homogenization using a high shear mixer.

Example 3A

| | |
|---|---|
| Oil (emollient) - tricaprin: | 26.7 |
| Starch - quinoa | 8.9 |
| Alcohol -ethanol | 43.6 |
| Denaturizing agent - MEK | 1.2 |
| Humectant | |
| Thickener - HPC | 0.9 |
| Water | 18.7 |
| Sum: | 100 |

Example 3B

| | |
|---|---|
| Oil (emollient) - triolein: | 27.0 |
| Starch - quinoa | 9.0 |

(continued)

| Alcohol -ethanol | 38.7 |
|---|---|
| Alcohol -isopropyl alcohol | 5.4 |
| Denaturizing agent - butanol | 0.01 |
| Humectant | |
| Thickener - HPC | 0.9 |
| Water | 19.0 |
| Sum: | 100 |

Example 3C

| Oil (emollient) - trieicosenoin: | 26.9 |
|---|---|
| Starch - quinoa | 9.0 |
| Alcohol -ethanol | 43.4 |
| Alcohol - n-propanol | 0.5 |
| Denaturizing agent: | |
| MEK | 0.5 |
| Bitrex | 10 ppm |
| Humectant | - |
| Thickener - Carbopol | 0.9 |
| Water | 18.8 |
| Sum: | 100 |

Example 3D

| Oil (emollient) - triolein: | 12.9 |
|---|---|
| Oil (emollient) - trilinolein: | 12.9 |
| Starch - quinoa | 8.6 |
| Alcohol -isopropyl alcohol | 36.2 |
| Denaturizing agent: | - |
| Humectant: glycerol | 4.3 |
| Thickener - HPC | 0.9 |
| Water | 24.2 |
| Sum: | 100 |

Example 3E

| Oil (emollient) - trierucin: | 25.9 |
|---|---|
| Starch - quinoa | 8.6 |
| Alcohol -n-propanol | 36.2 |
| Denaturizing agent: | - |
| Humectant: urea | 4.3 |
| Thickener - HPC | 0.9 |
| Water | 24.1 |
| Sum: | 100 |

Example 3F

| Oil (emollient) - MCT: | 25.6 |
|---|---|
| Starch - quinoa | 8.5 |
| Alcohol -isopropyl alcohol | 36.4 |
| Denaturizing agent: | - |
| Humectant: glycerol | 4.3 |
| Thickener - HPC | 0.9 |
| Water | 24.3 |
| Sum: | 100 |

Following Examples 3G - 3L are prepared as emollient hand sanitizers comprising oil/alcohol:water in an amount of 1/99 to 50/50 w/w. The compositions obtained are semisolid, liquid like.

Example 3G

| Oil (emollient) - trimyristin | 49.8 |
|---|---|
| Starch - quinoa | 16.6 |
| Alcohol -ethanol | 22.9 |
| Denaturizing agent: MEK | 0.7 |
| Humectant: glycerol | - |
| Thickener - HPC | - |
| Water | 10.0 |
| Sum: | 100 |

Example 3H

| Oil (emollient) - ttriolein | 48.2 |
|---|---|
| Starch - quinoa | 16.0 |
| Alcohol -ethanol | 35.1 |
| Denaturizing agent: MEK | 0.7 |
| Humectant: glycerol | - |
| Thickener - HPC | - |
| Water | 0 |
| Sum: | 100 |

Example 3I

| Oil (emollient) - MCT | 26.7 |
|---|---|
| Starch - quinoa | 8.9 |
| Alcohol -ethanol | 35.6 |
| Denaturizing agent: MEK | 1.2 |
| Humectant: glycerol | - |
| Thickener - HPC | 0.9 |
| Water | 26.7 |

(continued)

| | |
|---|---|
| Sum: | 100 |

Example 3J

| | |
|---|---|
| Oil (emollient) - triolein | 25.6 |
| Starch - quinoa | 8.5 |
| Alcohol -ethanol | 35.8 |
| Denaturizing agent: MEK | 1.2 |
| Humectant: glycerol | 4.3 |
| Thickener - Xanthan gum | 0.7 |
| Water | 23.9 |
| Sum: | 100 |

Example 3K

| | |
|---|---|
| Oil (emollient) - petrolatum | 9.5 |
| Starch - quinoa | 3.1 |
| Alcohol -ethanol | 59.8 |
| Denaturizing agent: MEK | - |
| Humectant: glycerol | - |
| Thickener - HPC | 1.9 |
| Water | 25.7 |
| Sum: | 100 |

Example 3L

| | |
|---|---|
| Oil (emollient) - trierucin | 1.2 |
| Starch - quinoa | 0.3 |
| Alcohol -ethanol | 55.7 |
| Denaturizing agent: MEK | - |
| Humectant: urea | 4.7 |
| Thickener - HPC | 0.9 |
| Water | 37.2 |
| Sum: | 100 |

Following Examples 3M -3R are prepared as emollient hand sanitizers Another aspect of the invention is a topical sanitizing composition comprising oil and alcohol+water in a ratio of 1/99 - 10/90 (w/w). Generally, such hand sanitizer comprises:

| | |
|---|---|
| Oil (emollient) | 1.0-10 |
| Starch | 0.3-3.5 |
| Alcohol (C1-C4 alcohol) | 50-70 |
| Denaturizing agent: | n.a. |
| Humectant: | (0-5) |
| Thickener | 0.2-1 |

(continued)

| | |
|---|---|
| Water | 20-40 |
| Sum: | 100 |

Example 3M

| | |
|---|---|
| Oil (emollient) -tricaprin | 4.8 |
| Starch - quinoa | 1.6 |
| Alcohol - ethanol | 63.9 |
| Denaturizing agent: MEK | 1.8 |
| Humectant: | - |
| Thickener: HPC | 0.5 |
| Water | 27.6 |
| Sum: | 100 |

Example 3N

| | |
|---|---|
| Oil (emollient) -triolein | 4.7 |
| Starch - quinoa | 1.6 |
| Alcohol - ethanol | 57.4 |
| Alcohol - isopropyl alcohol | 9.0 |
| Denaturizing agent: butanol | 0.01 |
| Humectant: | - |
| Thickener: HPC | 0.3 |
| Water | 27.1 |
| Sum: | 100 |

Example 3O

| | |
|---|---|
| Oil (emollient) -trieicosenoin | 4.8 |
| Starch - quinoa | 1.6 |
| Alcohol - ethanol | 59.6 |
| Alcohol - isopropyl alcohol | 8.5 |
| Denaturizing agent: butanol | 0.01 |
| Humectant: | - |
| Thickener: HPC | 0.2 |
| Water | 25.3 |
| Sum: | 100 |

Example 3P

| | |
|---|---|
| Oil (emollient) -triolein | 2.3 |
| Oil (emollient) -trilinolein | 2.3 |
| Starch - quinoa | 1.6 |

(continued)

| | |
|---|---|
| Alcohol - isopropyl alcohol | 56.0 |
| Denaturizing agent: butanol | - |
| Humectant: glycerol | |
| Thickener: HPC | 0.5 |
| Water | 37.3 |
| Sum: | 100 |

Example 3Q

| | |
|---|---|
| Oil (emollient) -trierucin | 4.7 |
| Starch - quinoa | 1.6 |
| Alcohol - isopropyl alcohol | 53.2 |
| Denaturizing agent: butanol | - |
| Humectant: urea | 4.7 |
| Thickener: HPC | 0.5 |
| Water | 35.3 |
| Sum: | 100 |

Example 3R

| | |
|---|---|
| Oil (emollient) -tricaprin | 4.6 |
| Starch - quinoa | 1.6 |
| Alcohol - isopropyl alcohol | 61.1 |
| Denaturizing agent: MEK | 1.6 |
| Humectant: urea | 4.6 |
| Thickener: HPC | 0.5 |
| Water | 26.0 |
| Sum: | 100 |

Example 4A-G.

Ex vivo Perception Testing utilizing ForceBoard™ with VITRO-SKIN®

Example 4A - Reference product (provided by Sterisol)

| | |
|---|---|
| Alcohol - ethanol | 70 |
| Alcohol - isopropyl alcohol | 10 |
| Water | 20 |
| Sum: | 100 |

Example 4B

| | |
|---|---|
| Oil (emollient) -tricaprin | 0 |
| Starch - quinoa | 10 |

(continued)

| | |
|---|---|
| Alcohol - ethanol | 60 |
| Water | 60 |
| Sum: | 100 |

Example 4C

| | |
|---|---|
| Oil (emollient) -tricaprin | 30 |
| Starch - quinoa | 10 |
| Alcohol - ethanol | 33 |
| Water | 27 |
| Sum: | 100 |

Example 4D

| | |
|---|---|
| Oil (emollient) -tricaprin | 30 |
| Starch - quinoa | 10 |
| Alcohol - ethanol | 42 |
| Water | 18 |
| Sum: | 100 |

Example 4E

| | |
|---|---|
| Oil (emollient) -triolein | 0 |
| Starch - quinoa | 10 |
| Alcohol - ethanol | 60 |
| Water | 60 |
| Sum: | 100 |

Example 4F

| | |
|---|---|
| Oil (emollient) -triolein | 30 |
| Starch - quinoa | 10 |
| Alcohol - ethanol | 33 |
| Water | 27 |
| Sum: | 100 |

Example 4G

| | |
|---|---|
| Oil (emollient) -triolein | 30 |
| Starch - quinoa | 10 |
| Alcohol - ethanol | 42 |
| Water | 18 |

(continued)

| Sum: | 100 |
|---|---|

Example 4H Emollient handsanitizer gel

| Oil (emollient) - triolein: | 5 |
|---|---|
| Starch - modified quinoa | 1.95 |
| Alcohol -ethanol | 55.1 |
| Humectant -glycerin | 1 |
| Thickener - Carbomer | 0.25 |
| Amino methyl propyl (AMP) | 0.11 |
| Water | 36.69 |
| Sum: | 100 |

Example 4I Antimicrobial cream

| Oil (emollient) - triolein: | 28 |
|---|---|
| Starch - modified quinoa | 11 |
| Alcohol -ethanol | 34.4 |
| Humectant -glycerin | 1 |
| Thickener - Carbomer | 0.25 |
| Water | 36.69 |
| Sum: | 100 |

Example 4J. In vivo Perception Testing - panel with volunteers.

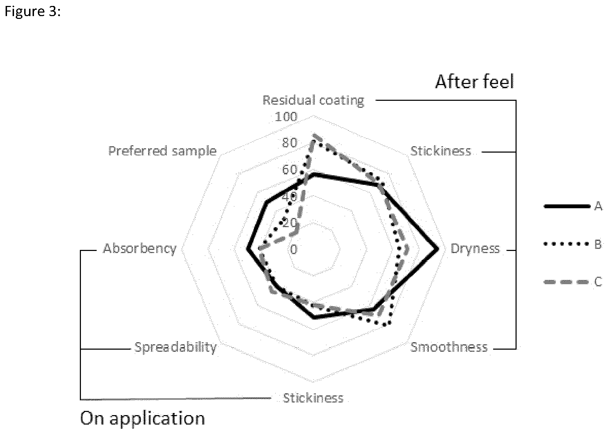

A sensory evaluation was performed as a blind ranking test including three samples; i.e. a Pickering formulation (A), comprising 20% oil in ethanol/water 70/30 (by weight), and two classical hand sanitizers, Sterisol® Handdesinfektion Etanol (Sterisol AB, Sweden) (B) and DAX Clinical Handdesinfektion (CCS Healthcare AB, Sweden). The panel included 29 volunteers. Equal amounts of each sample was applied on a marked area on the palm of the hand. Properties were evaluated on application (i.e., spreadability, absorbency, and stickiness) and after application (i.e., after feeel in terms of residual coating, dryness, smoothness and stickiness). The participants of the panel were also asked to rate the products with respect to their preferences. From the results summarized in figure 3 it is evident that the two classical handsanitizers came out very similar in this test, while the Pickering formulation had a more dry after feel and gave the perception of less residual coating. Perception of less residual coating on the skin is good, but surprisingly, due to the fact that the part of oil and solid particles in the pickering formulations stays on the skin (provides emollient properties) while most of the ingredients included in the classical handsanitizers, comprising mainly alcohol and water, will evaporate during application.

The pickering formulation was also deemed as the most prefered product of the three.

Example 5: Antimicrobial efficacy

The antimicrobial efficacy of the compositions was tested following the standard method EN 13727, Bacterial efficacy in suspension test (log10 RF $\geq$ 5) ("Chemical Disinfectants and antiseptics: Quantitative Suspension Test for the Evaluation of Bactericidal Activity for Instruments Used in the Medical Area")

Further standard methods are designed to evaluate bactericidal, fungicidal, yeasticidal, basic sporicidal, or mycobactericidal activity of a product used under various conditions. Examples of standard methods are EN 13727 for testing bacteria, EN 13624 for testing yeast (such as Candida albicans), EN 14476 for virus test, and EN 14348 for test of bacteria, like tuberculocidal bacteria.

Standard methods are also designed for different applications, for example EN 1500 for Hygienic hand disinfection, and EN 12791 for surgical hand disinfection.

Herein, the test organism is exposed to hand sanitizer compositions as defined herein in a manner which simulates the desired claim. Following exposure, the test system is neutralized and quantitatively assayed for survivors. The plates are incubated, enumerated, and a reduction in viability or microbiocidal effect is determined as compared to a population control.

Following organisms are included in the test:

*Staphylococcus aureus (tested)*
*Escherichia coli K12 (tested)*
*Escherichia hirae*
*Pseudomyses aeruginosa*

Typical performance criteria (Requirements may vary by claim): 3-5 log reduction in 1-5 minutes depending on claim. For hygienic handwashing it is 3 log, for other performances 5 log, during 30-60 seconds (mandatory for hygienic handwashing and hand rub).

Example 5A

Method EN 13727 (Bactericidal efficacy in suspension test, $\log_{10}$ RF $\geq$ 5), *Escherichia coli* K12 and *Staphylococcus aureus,* Dilution to 80%.

| Formulation | E. coli / $\log_{10}$ RF | S. aureus / $\log_{10}$ RF |
|---|---|---|
| | 80% | 80% |
| 58% EtOH (aq) | > 5.1 | > 5.3 |
| 58% EtOH (aq) | > 5.4 | > 5.3 |
| 42% EtOH (aq) | 6.4 | 4.8 |
| 35% EtOH (aq) | > 5.1 | <2.7 |
| 35% EtOH (aq) | > 5.4 | 3 |
| | | |
| Pickering 42% EtOH | 6.3 | 6.4 |
| Pickering 35% EtOH | 4.8 | > 5.2 |
| Pickering 35% EtOH | > 5.4 | > 5.3 |
| Pickering 0% EtOH | < 2.4 | <2.7 |
| Pickering 0% EtOH | <2.7 | <2.6 |

References:

[1] *(Eiteman et al., 1994; McClements, 2015)*
[2] *Skedung et al., 2013*

## Claims

1. An oil-in-water emulsion composition comprising

a) 40-98% of at least one liquid continuous aqueous phase (X) comprising at least one linear or branched $C_1$-$C_4$ alcohol selected from the group consisting of ethanol, isopropyl alcohol, and n-propanol;
b) 1-40% of at least one dispersed fatty phase (Y), comprising at least one oil having biological origin, selected from triglyceride oils comprising fatty acids selected from myristic acid, palmitic acid, stearic acid, oleic acid, linolic acid, linolenic acid, pelargonic acid, capric acid, caprylic acid, lauric acid, or mixture thereof;
c) 0.25 - 20% of stabilizing particles (Z), consisting of components being solid particles; wherein the emulsion is a Pickering emulsion; wherein the emulsion is surfactant free; wherein the emulsion composition comprises at least 20%, by weight, of said $C_1$-$C_4$ alcohol, and wherein the dispersed fatty phase is selected to have no, or at least very limited solubility in the continuous aqueous phase (X); and wherein the solid particles (Z) consists of starch.

2. The oil-in-water emulsion composition according to claim 1 wherein the emulsion composition comprises at least 35 % by weight of C1-C4 alcohol.

3. The oil-in-water emulsion according to any of claims 1 and 2, wherein the at least one dispersed fatty phase (Y) have a melting point below 50ºC, typically below 37ºC, typically between 0 and 32ºC.

4. The emulsion composition according to any of claims 1 to 3, wherein emulsion composition comprises between 40 and 80% $C_1$-$C_4$ alcohol as the at least one liquid continuous phase; preferably 40-70% $C_1$-$C_4$ alcohol.

5. The emulsion composition according to any of claims 1 to 4, wherein the at least one dispersed fatty phase (Y) is in solid or liquid phase; preferably wherein the solid phase is crystalline.

6. The emulsion composition according to any of claims 1 to 4, wherein the at least one dispersed fatty phase (Y) is in solid or liquid phase; preferably wherein the solid phase is amorphous.

7. The emulsion composition according to any of claims 1 to 6, wherein the at least one dispersed fatty phase is a mixture of the oil having biological origin, selected from triglyceride oils; wherein the triglyceride comprises fatty acids selected from myristic acid, palmitic acid, stearic acid, oleic acid, linolic acid, linolenic acid, pelargonic acid, capric acid, caprylic acid, lauric acid, or mixture thereof; and mineral oil.

8. The emulsion composition according to any of claims 1 to 7, wherein the solid particles have a size of 0.005-20 $\mu$m; typically a size of 0.05-20 $\mu$m.

9. The emulsion composition according to any of claims 1 to 8, wherein the starch is obtained from any botanical source.

10. The emulsion composition according to any of claims 1 to 9, wherein the ratio of b) at least one dispersed fatty phase (Y), and the c) stabilizing particles (Z), is between 2.5:1 to 5:1.

11. The emulsion composition according to any of claims 1 to 10, wherein the emulsion composition also comprises one or more additives.

12. A topical sanitizing composition comprising the oil-in-water emulsion composition according to any of claims 1 to 11.

13. The topical sanitizing composition according to claim 12 wherein the composition is in form of a cream; in form of a foam; in form of a gel; in form of a lotion; or in form of a solution.

14. The topical sanitizing composition according to any of claims 12 and 13, wherein the composition has an antimicrobial effect; preferably the composition has an effect upon Gram negative bacteria, Gram positive bacteria, yeast organisms, and enveloped viruses.

15. A method for preparing the emulsion composition according to any of claims 1 to 11, wherein the method comprises the steps:

a) providing at least one liquid continous aqueous phase (X) comprising at least one linear or branched $C_1$-$C_4$-alcohol;
b) providing at least one dispersed fatty phase (Y), comprising at least one oil; c) providing stabilizing particles (Z);
d1) dispersing the stabilizing particles (Z) in the continuous aqueous phase (X) of a) during mixing, or
d2) dispersing the stabilizing particles (Z) in the dispersed fatty phase (Y);
e) adding the dispersed fatty phase (Y) to the mixture (X+Z) of d1), or adding the continuous aqueous phase (X) to the mixture (Y+Z) of d2), during mixing, at a temperature below 45ºC;
f) optionally, emulsification by further mixing; and
g) optionally addition of additives during or after any of the steps a) to e).

16. A method for preparing a topical sanitizing composition as defined in any of claims 12 to 14, comprising the steps:

a) providing at least one liquid continous aqueous phase (X) comprising at least one linear or branched $C_1$-$C_4$-alcohol;

b) providing at least one dispersed fatty phase (Y), comprising at least one oil; c) providing stabilizing particles (Z);

d1) dispersing the stabilizing particles (Z) in the continuous aqueous phase (X) of a) during mixing, or

d2) dispersing the stabilizing particles (Z) in the dispersed fatty phase (Y);

e) adding the dispersed fatty phase (Y) to the mixture (X+Z) of d1), or adding the continuous aqueous phase (X) to the mixture (Y+Z) of d2), during mixing, at a temperature below 45ᴼC;

f) optionally, emulsification by further mixing; and

g) optionally addition of additives selected from antimicrobial agent, denaturing agent, thickener, humectants, antipruritic agent, fragrances, essential oils, propellants, during or after any of the steps a) to e).

**Patentansprüche**

1. Öl-in-Wasser-Emulsionszusammensetzung, umfassend

a) 40-98 % mindestens einer flüssigen kontinuierlichen Wasserphase (X), die mindestens einen linearen oder verzweigten $C_1$-$C_4$-Alkohol ausgewählt aus der Gruppe bestehend aus Ethanol, Isopropylalkohol und n-Propanol umfasst;

b) 1-40 % mindestens einer dispergierten Fettphase (Y), die mindestens ein Öl biologischer Herkunft umfasst, das aus Triglyceridölen, die aus Myristinsäure, Palmitinsäure, Stearinsäure, Ölsäure, Linolsäure, Linolensäure, Pelargonsäure, Caprinsäure, Caprylsäure, Laurinsäure oder einer Mischung davon ausgewählte Fettsäuren umfassen, ausgewählt ist;

c) 0,25-20 % stabilisierende Teilchen (Z), bestehend aus Komponenten, bei denen es sich um feste Teilchen handelt; wobei es sich bei der Emulsion um eine Pickering-Emulsion handelt; wobei die Emulsion tensidfrei ist; wobei die Emulsionszusammensetzung mindestens 20 Gew.-% des $C_1$-$C_4$-Alkohols umfasst und wobei die dispergierte Fettphase so gewählt ist, dass sie keine oder zumindest eine sehr begrenzte Löslichkeit in der kontinuierlichen Wasserphase (X) aufweist; und wobei die festen Teilchen (Z) aus Stärke bestehen.

2. Öl-in-Wasser-Emulsionszusammensetzung nach Anspruch 1, wobei die Emulsionszusammensetzung mindestens 35 Gew.-% des $C_1$-$C_4$-Alkohols umfasst.

3. Öl-in-Wasser-Emulsion nach einem der Ansprüche 1 und 2, wobei die mindestens eine dispergierte Fettphase (Y) einen Schmelzpunkt unter 50 °C, typischerweise unter 37 °C, typischerweise zwischen 0 und 32 °C, aufweist.

4. Emulsionszusammensetzung nach einem der Ansprüche 1 bis 3, wobei die Emulsionszusammensetzung zwischen 40 und 80 % $C_1$-$C_4$-Alkohol als die mindestens eine flüssige kontinuierliche Phase, vorzugsweise 40-70 % $C_1$-$C_4$-Alkohol, umfasst.

5. Emulsionszusammensetzung nach einem der Ansprüche 1 bis 4, wobei die mindestens eine dispergierte Fettphase (Y) in fester oder flüssiger Phase vorliegt, vorzugsweise wobei die feste Phase kristallin ist.

6. Emulsionszusammensetzung nach einem der Ansprüche 1 bis 4, wobei die mindestens eine dispergierte Fettphase (Y) in fester oder flüssiger Phase vorliegt, vorzugsweise wobei die feste Phase amorph ist.

7. Emulsionszusammensetzung nach einem der Ansprüche 1 bis 6, wobei es sich bei der mindestens einen dispergierten Fettphase um eine Mischung von dem Öl biologischer Herkunft, das aus Triglyceridölen ausgewählt ist; wobei das Triglycerid aus Myristinsäure, Palmitinsäure, Stearinsäure, Ölsäure, Linolsäure, Linolensäure, Pelargonsäure, Caprinsäure, Caprylsäure, Laurinsäure oder einer Mischung davon ausgewählte Fettsäuren umfasst, ausgewählt ist; und Mineralöl handelt.

8. Emulsionszusammensetzung nach einem der Ansprüche 1 bis 7, wobei die festen Teilchen eine Größe von 0,005-20 μm, typischerweise eine Größe von 0,05-20 μm, aufweisen.

9. Emulsionszusammensetzung nach einem der Ansprüche 1 bis 8, wobei die Stärke aus einer beliebigen botanischen Quelle erhalten wird.

**10.** Emulsionszusammensetzung nach einem der Ansprüche 1 bis 9, wobei das Verhältnis von b) mindestens einer dispergierten Fettphase (Y) und den c) stabilisierenden Teilchen (Z) zwischen 2,5:1 bis 5:1 liegt.

**11.** Emulsionszusammensetzung nach einem der Ansprüche 1 bis 10, wobei die Emulsionszusammensetzung außerdem ein oder mehrere Additive umfasst.

**12.** Topische Desinfektionszusammensetzung, umfassend die Öl-in-Wasser-Emulsionszusammensetzung nach einem der Ansprüche 1 bis 11.

**13.** Topische Desinfektionszusammensetzung nach Anspruch 12, wobei die Zusammensetzung in Form einer Creme, in Form eines Schaums, in Form eines Gels, in Form einer Lotion oder in Form einer Lösung vorliegt.

**14.** Topische Desinfektionszusammensetzung nach einem der Ansprüche 12 und 13, wobei die Zusammensetzung eine antimikrobielle Wirkung aufweist; vorzugsweise die Zusammensetzung eine Wirkung auf gramnegative Bakterien, grampositive Bakterien, Hefeorganismen und behüllte Viren aufweist.

**15.** Verfahren zur Herstellung der Emulsionszusammensetzung nach einem der Ansprüche 1 bis 11, wobei das Verfahren folgende Schritte umfasst:

> a) Bereitstellen mindestens einer flüssigen kontinuierlichen Wasserphase (X), die mindestens einen linearen oder verzweigten $C_1$-$C_4$-Alkohol umfasst;
> b) Bereitstellen mindestens einer dispergierten Fettphase (Y), die mindestens ein Öl umfasst;
> c) Bereitstellen von stabilisierenden Teilchen (Z);
> d1) Dispergieren der stabilisierenden Teilchen (Z) in der kontinuierlichen Wasserphase (X) aus a) unter Mischen oder
> d2) Dispergieren der stabilisierenden Teilchen (Z) in der dispergierten Fettphase (Y);
> e) Zugeben der dispergierten Fettphase (Y) zu der Mischung (X+Z) aus d1) oder Zugeben der kontinuierlichen Wasserphase (X) zu der Mischung (Y+Z) aus d2) unter Mischen bei einer Temperatur unter 45 °C;
> f) gegebenenfalls Emulgieren durch weiteres Mischen und
> g) gegebenenfalls Zugeben von Additiven während oder nach einem der Schritte a) bis e).

**16.** Verfahren zur Herstellung einer topischen Desinfektionszusammensetzung gemäß einem der Ansprüche 12 bis 14, das folgende Schritte umfasst:

> a) Bereitstellen mindestens einer flüssigen kontinuierlichen Wasserphase (X), die mindestens einen linearen oder verzweigten $C_1$-$C_4$-Alkohol umfasst;
> b) Bereitstellen mindestens einer dispergierten Fettphase (Y), die mindestens ein Öl umfasst;
> c) Bereitstellen von stabilisierenden Teilchen (Z);
> d1) Dispergieren der stabilisierenden Teilchen (Z) in der kontinuierlichen Wasserphase (X) aus a) unter Mischen oder
> d2) Dispergieren der stabilisierenden Teilchen (Z) in der dispergierten Fettphase (Y);
> e) Zugeben der dispergierten Fettphase (Y) zu der Mischung (X+Z) aus d1) oder Zugeben der kontinuierlichen Wasserphase (X) zu der Mischung (Y+Z) aus d2) unter Mischen bei einer Temperatur unter 45 °C;
> f) gegebenenfalls Emulgieren durch weiteres Mischen und
> g) gegebenenfalls Zugeben von Additiven, die aus einem antimikrobiellen Mittel, einem Denaturierungsmittel, einem Verdicker, Feuchthaltemitteln, einem Mittel gegen Juckreiz, Duftstoffen, ätherischen Ölen und Treibmitteln ausgewählt werden, während oder nach einem der Schritte a) bis e) .

**Revendications**

**1.** Composition d'émulsion huile-dans-eau comprenant

> a) 40 à 98 % d'au moins une phase aqueuse continue liquide (X) comprenant au moins un alcool linéaire ou ramifié en $C_1$-$C_4$ choisi dans le groupe constitué par l'éthanol, l'alcool isopropylique et le n-propanol ;
> b) 1 à 40 % d'au moins une phase graisseuse dispersée (Y), comprenant au moins une huile ayant une origine biologique, choisie parmi des huiles de triglycérides comprenant des acides gras choisis parmi l'acide myristique, l'acide palmitique, l'acide stéarique, l'acide oléique, l'acide linoléique, l'acide linolénique, l'acide pélargonique, l'acide caprique, l'acide caprylique, l'acide laurique ou un mélange correspondant ;

c) 0,25 à 20 % de particule stabilisantes (Z), constituées de composants qui sont des particules solides ; l'émulsion étant une émulsion Pickering ; l'émulsion étant exempte de tensioactif ; la composition d'émulsion comprenant au moins 20 %, en poids dudit alcool en $C_1$-$C_4$, et la phase graisseuse dispersée étant choisie pour avoir aucune solubilité ou une solubilité au moins très limitée dans la phase aqueuse continue (X) ; et les particules solides (Z) étant constituées d'amidon.

2. Composition d'émulsion huile-dans-eau selon la revendication 1, la composition d'émulsion comprenant au moins 35 % en poids d'alcool en $C_1$-$C_4$.

3. Composition d'émulsion huile-dans-eau selon l'une quelconque des revendications 1 et 2, l'au moins une phase graisseuse dispersée (Y) possédant un point de fusion inférieur à 50 °C, généralement inférieur à 37 °C, généralement entre 0 et 32 °C.

4. Composition d'émulsion selon l'une quelconque des revendications 1 à 3, la composition d'émulsion comprenant entre 40 et 80 % d'alcool en $C_1$-$C_4$ en tant que l'au moins une phase continue liquide ; préférablement 40 à 70 % d'alcool en $C_1$-$C_4$.

5. Composition d'émulsion selon l'une quelconque des revendications 1 à 4, l'au moins une phase graisseuse dispersée (Y) étant en phase solide ou liquide ; préférablement la phase solide étant cristalline.

6. Composition d'émulsion selon l'une quelconque des revendications 1 à 4, l'au moins une phase graisseuse dispersée (Y) étant en phase solide ou liquide ; préférablement la phase solide étant amorphe.

7. Composition d'émulsion selon l'une quelconque des revendications 1 à 6, l'au moins une phase graisseuse dispersée étant un mélange de l'huile ayant une origine biologique, choisie parmi des huiles de triglycérides ; le triglycéride comprenant des acides gras choisis parmi l'acide myristique, l'acide palmitique, l'acide stéarique, l'acide oléique, l'acide linoléique, l'acide linolénique, l'acide pélargonique, l'acide caprique, l'acide caprylique, l'acide laurique ou un mélange correspondant ; et d'une huile minérale.

8. Composition d'émulsion selon l'une quelconque des revendications 1 à 7, les particules solides possédant une taille de 0,005 à 20 μm ; généralement une taille de 0,05 à 20 pm.

9. Composition d'émulsion selon l'une quelconque des revendications 1 à 8, l'amidon étant obtenu d'une quelconque source botanique.

10. Composition d'émulsion selon l'une quelconque des revendications 1 à 9, le rapport de b) au moins une phase graisseuse dispersée (Y), et des c) particules stabilisantes (Z) étant compris entre 2,5 : 1 et 5 : 1.

11. Composition d'émulsion selon l'une quelconque des revendications 1 à 10, la composition d'émulsion comprenant également un ou plusieurs additifs.

12. Composition désinfectante topique comprenant la composition d'émulsion huile-dans-eau selon l'une quelconque des revendications 1 à 11.

13. Composition désinfectante topique selon la revendication 12, la composition étant sous forme d'une crème ; sous forme d'une mousse ; sous forme d'un gel ; sous forme d'une lotion ; ou sous forme d'une solution.

14. Composition désinfectante topique selon l'une quelconque des revendications 12 et 13, la composition possédant un effet antimicrobien ; préférablement la composition possédant un effet sur des bactéries Gram-négatives, des bactéries Grampositives, des organismes de type levure et des virus enveloppés.

15. Procédé pour la préparation de la composition d'émulsion selon l'une quelconque des revendications 1 à 11, le procédé comprenant les étapes :

a) fourniture d'au moins une phase aqueuse continue liquide (X) comprenant au moins un alcool linéaire ou ramifié en $C_1$-$C_4$ ;

b) fourniture d'au moins une phase graisseuse dispersée (Y), comprenant au moins une huile ;

c) fourniture de particules stabilisantes (Z) ;

d1) dispersion des particules stabilisantes (Z) dans la phase aqueuse continue (X) de a) pendant un mélange, ou

d2) dispersion des particules stabilisantes (Z) dans la phase graisseuse dispersée (Y) ;

e) ajout de la phase graisseuse dispersée (Y) au mélange (X + Z) de d1), ou ajout de la phase aqueuse continue (X) au mélange (Y + Z) de d2), pendant un mélange, à une température inférieure à 45 °C ;

f) éventuellement, émulsification en mélangeant davantage ; et

g) éventuellement ajout d'additifs pendant ou après l'une quelconque parmi les étapes a) à e).

16. Procédé pour la préparation d'une composition désinfectante topique telle que définie dans l'une quelconque des revendications 12 à 14, comprenant les étapes :

a) fourniture d'au moins une phase aqueuse continue liquide (X) comprenant au moins un alcool linéaire ou ramifié en $C_1$-$C_4$ ;

b) fourniture d'au moins une phase graisseuse dispersée (Y), comprenant au moins une huile ;

c) fourniture de particules stabilisantes (Z) ;

d1) dispersion des particules stabilisantes (Z) dans la phase aqueuse continue (X) de a) pendant un mélange, ou

d2) dispersion des particules stabilisantes (Z) dans la phase graisseuse dispersée (Y) ;

e) ajout de la phase graisseuse dispersée (Y) au mélange (X + Z) de d1), ou ajout de la phase aqueuse continue (X) au mélange (Y + Z) de d2), pendant un mélange, à une température inférieure à 45 °C ;

f) éventuellement, émulsification en mélangeant davantage ; et

g) éventuellement ajout d'additifs choisis parmi un agent antimicrobien, un agent dénaturant, un épaississant, des humectants, un agent antiprurigineux, des fragrances, des huiles essentielles, des agents propulseurs, pendant ou après l'une quelconque parmi les étapes a) à e).

Figure 1:

Figure 1A

Figure 1B-a

Figure 1B-b

Figure 1C

Figure 1D-a

Figure 1D-b

Figure 1D-c

Figure 1 E

Figure 2A:

Figure 2B:

Figure 2C:

Figure 2D:

Figure 3:

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009109870 A
- WO 2012082065 A1
- US 2004241120 A1
- US 2009226498 A1
- EP 3167871 A
- WO 2012082065 A

**Non-patent literature cited in the description**

- **EITEMAN et al.** *McClements,* 1994